# EUROPEAN PATENT SPECIFICATION

(11) **EP 3 508 642 B1**
(45) Date of publication and mention of the grant of the patent: **15.01.2020**
(21) Application number: 16915116.4
(22) Date of filing: 31.08.2016
(51) Int. Cl.: D06C 29/00, A61F 13/15

(54) **METHOD AND DEVICE FOR PRODUCING SHEET-LIKE MEMBER FOR ABSORBENT ARTICLE**
VERFAHREN UND VORRICHTUNG ZUR HERSTELLUNG EINES FLÄCHENFÖRMIGEN ELEMENTS FÜR EINEN SAUGFÄHIGEN ARTIKEL
PROCÉDÉ ET DISPOSITIF DE FABRICATION D'ÉLÉMENT DE TYPE FEUILLE POUR ARTICLE ABSORBANT

(43) Date of publication of application: 10.07.2019
(73) Proprietor: Unicharm Corporation, Shikokuchuo-shi, Ehime 799-0111 (JP)
(72) Inventor: ISHIKAWA, Shinichi, Kanonji-shi Kagawa 769-1602 (JP); HANO, Shodai, Kanonji-shi Kagawa 769-1602 (JP); ISHIKAWA, Yoshihide, Kanonji-shi Kagawa 769-1602 (JP)
(74) Representative: Staeger & Sperling Partnerschaftsgesellschaft mbB
(86) International application number: PCT/JP2016/075491
(87) International publication number: WO 2018/042555

(56) References cited:
- WO-A1-96/10481
- JP-A- H01 256 956
- JP-A- H08 299 725
- JP-A- H11 189 960
- JP-A- 2007 126 766
- JP-A- 2009 513 298
- US-A1- 2003 121 380
- US-A1- 2011 201 243

## Description

### [Technical Field]

The present invention relates to a method and a device for manufacturing a sheet-like member for an absorbent article such as a disposable diaper.

### [Background Art]

Conventionally, in a manufacturing line for an absorbent article such as a disposable diaper that absorbs excrement such as urine, there are cases where processing for forming through holes in a continuous sheet that is continuous in the direction of transport is performed for the purpose of giving breathability to the absorbent article. As one example of a method for forming such through holes, PTL 1 discloses that press-in members, which project from the outer circumferential surface of a rotating roller, are pressed in the thickness direction into the continuous sheet.

### [Citation List]

### [Patent Literature]

[PTL 1] Japanese Patent Application Publication No. 2003-171866 WO 96/10481 discloses a slit elastic nonwoven laminate and methods for making the same

### [Summary of Invention]

### [Technical Problem]

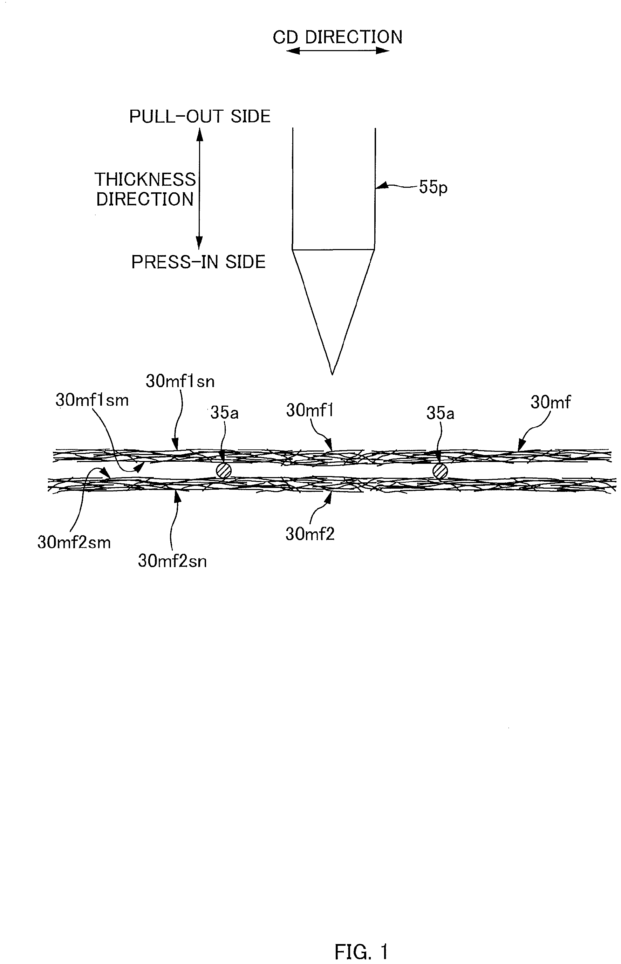

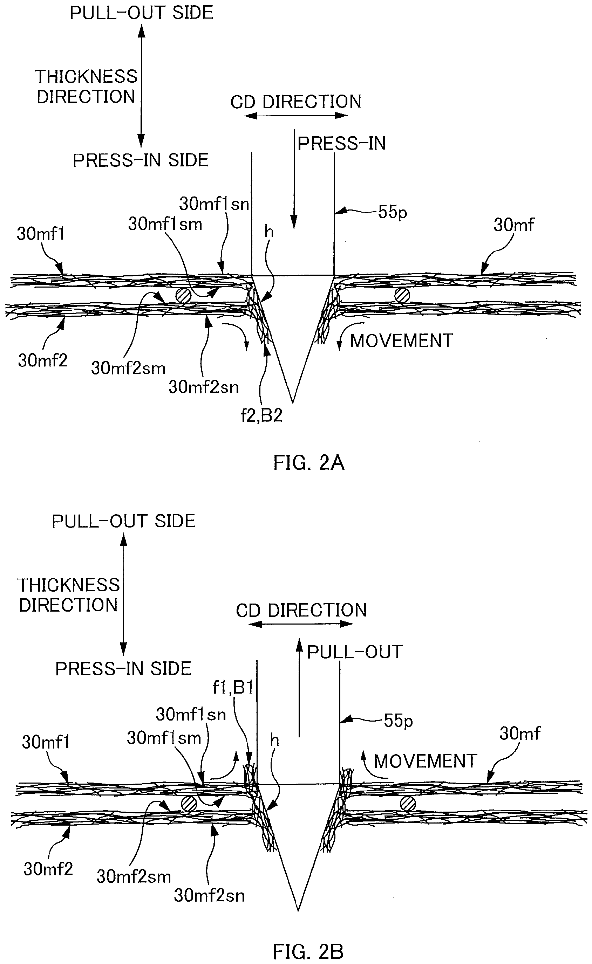

In this manufacturing line, it is also conceivable that through holes h are formed in a composite sheet 30mf such as the following. Specifically, first, the composite sheet 30mf, which is shown in a longitudinal schematic cross-sectional view in FIG. 1, has a first nonwoven fabric sheet 30mf1 that is continuous in a direction of transport (direction passing through the paper surface in FIG. 1), and a second nonwoven fabric sheet 30mf2 that is continuous in the direction of transport and arranged so as to overlap the first nonwoven fabric sheet 30mf1 in the thickness direction of the composite sheet 30mf. Also, multiple elastic members 35a, which extend in the direction of transport and are stretched in the direction of transport, are inserted between the first nonwoven fabric sheet 30mf1 and the second nonwoven fabric sheet 30mf2 in a side-by-side arrangement in a CD direction that intersects the direction of transport, and are fixed to the first nonwoven fabric sheet 30mf1 and the second nonwoven fabric sheet 30mf2 with use of an adhesive. When the through holes h are to be formed in this composite sheet 30mf, as shown in FIG. 2A, first, pin members 55p serving as the aforementioned press-in members are pressed into the composite sheet 30mf from the side corresponding to a non-facing surface 30mflsn, which is the one of two surfaces 30mflsn and 30mflsm of the first nonwoven fabric sheet 30mf1 in the thickness direction that does not face the second nonwoven fabric sheet 30mf2. After being pressed in, as shown in FIG. 2B, the pin members 55p are pulled out toward the non-facing surface side 30mflsn, and when they are pulled out, fibers f1 clinging to the pin members 55p move together with the pin members 55p in the direction of pull-out. Accordingly, burrs (flip-ups) B1 that project outward in the thickness direction, which is the direction of pull-out, are formed on the non-facing surface 30mflsn of the first nonwoven fabric sheet 30mf1. If the burrs B1 are large, they may be detrimental to the texture and appearance of the absorbent article that is ultimately manufactured.

The present invention was achieved in light of conventional problems such as that described above, and an object of the present invention is to reduce the size of burrs that are formed on a non-facing surface of a first nonwoven fabric sheet, which is the one of the two surfaces thereof that does not face a second nonwoven fabric sheet, when press-in members such as pin members are pressed into a composite sheet from the non-facing surface side and then pulled out toward the non-facing surface in order to form through holes in the composite sheet.

### [Solution to Problem]

A main aspect of the present invention for achieving the above-described aspect is a method for manufacturing a sheet-like member for an absorbent article,
the sheet-like member being formed from a composite sheet that is continuous in a predetermined direction,
the sheet-like member being provided with a plurality of through holes,
the method including:
a transporting step of transporting the composite sheet in a direction of transport that is the predetermined direction,
   the composite sheet being constituted by a first nonwoven fabric sheet, a second nonwoven fabric sheet, and a plurality of elastic members,
   the first nonwoven fabric sheet being continuous in the predetermined direction,
   the second nonwoven fabric sheet being continuous in the predetermined direction and arranged overlapped with the first nonwoven fabric sheet in a thickness direction of the composite sheet,
   the plurality of elastic members being stretched in the predetermined direction and extending along the predetermined direction,
   the plurality of elastic members being inserted between and fixed to the first nonwoven fabric sheet and the second nonwoven fabric sheet in a side-by-side arrangement in a CD direction that intersects the predetermined direction; and
a through-hole forming step of forming the through holes by pressing a press-in member in the thickness direction at positions between a pair of elastic members that are adjacent in the CD direction in the composite sheet,
   the through holes penetrating the composite sheet in the thickness direction,
in the through-hole forming step,
   the press-in member being pressed in the thickness direction into a non-facing surface of the first nonwoven fabric sheet, and thereafter the press-in member being pulled out toward the non-facing surface,
   the non-facing surface being one of two surfaces of the first nonwoven fabric sheet,
   the non-facing surface not facing the second nonwoven fabric sheet,
   the non-facing surface of the first nonwoven fabric sheet being a surface that comes into contact with a support surface of a support member when constituent fibers of the first nonwoven fabric sheet are suctioned to and deposited on the support surface in order to produce the first nonwoven fabric sheet.

Further, a device for manufacturing a sheet-like member for an absorbent article,
the sheet-like member being formed from a composite sheet that is continuous in a predetermined direction,
the sheet-like member being provided with a plurality of through holes,
the device including:
a transporting device that transports the composite sheet in a direction of transport that is the predetermined direction,
   the composite sheet being constituted by a first nonwoven fabric sheet, a second nonwoven fabric sheet, and a plurality of elastic members,
   the first nonwoven fabric sheet being continuous in the predetermined direction,
   the second nonwoven fabric sheet being continuous in the predetermined direction and arranged overlapped with the first nonwoven fabric sheet in a thickness direction of the composite sheet,
   the plurality of elastic members being stretched in the predetermined direction and extending along the predetermined direction,
   the plurality of elastic members being inserted between and fixed to the first nonwoven fabric sheet and the second nonwoven fabric sheet in a side-by-side arrangement in a CD direction that intersects the predetermined direction; and
a through-hole forming device that forms the through holes by pressing a press-in member in the thickness direction at positions between a pair of elastic members that are adjacent in the CD direction in the composite sheet,
   the through holes penetrating the composite sheet in the thickness direction,
in the through-hole forming device,
   the press-in member being pressed in the thickness direction into a non-facing surface of the first nonwoven fabric sheet, and thereafter the press-in member being pulled out toward the non-facing surface,
   the non-facing surface being one of two surfaces of the first nonwoven fabric sheet,
   the non-facing surface not facing the second nonwoven fabric sheet,
   the non-facing surface of the first nonwoven fabric sheet being a surface that comes into contact with a support surface of a support member when constituent fibers of the first nonwoven fabric sheet are suctioned to and deposited on the support surface in order to produce the first nonwoven fabric sheet.

Features of the present invention other than the above will become clear by reading the description of the present specification with reference to the accompanying drawings.

### [Advantageous Effects of Invention]

According to the present invention, it is possible to reduce the size of burrs that are formed on a non-facing surface of a first nonwoven fabric sheet, which is the one of the two surfaces thereof that does not face a second nonwoven fabric sheet, when press-in members such as pin members are pressed into a composite sheet from the non-facing surface side and then pulled out toward the non-facing surface in order to form through holes in the composite sheet.

### [Brief Description of the Drawings]

FIG. 1 is a schematic diagram that illustrates processing for forming through holes h in a composite sheet 30mf.
FIGS. 2A and 2B are schematic diagrams that illustrate the same as above.
FIG. 3 is a schematic perspective view of a pants-shaped state of a three-piece type of disposable diaper 1, which is one example of an absorbent article, as viewed from a front side.
FIG. 4 is a schematic plan view of the diaper 1 in an unfolded state, as viewed from a skin side.
FIG. 5 is a cross-sectional view taken along V-V in FIG 4.
FIG. 6 is a schematic plan view of a front band member 31, as viewed from a non-skin side in a thickness direction.
FIG. 7 is a schematic enlarged view of air holes h according to an embodiment of the present invention, as viewed from the non-skin side in the thickness direction.
FIG. 8 is a schematic plan view of a manufacturing line for illustrating various types of processing performed in the manufacturing line.
FIG. 9A is a schematic plan view of an intermediate product 1m immediately before being transported to an air-hole forming step S50 position.
FIG. 9B is a schematic plan view of an intermediate product 1m after passing through the air-hole forming step S50.
In FIG. 10, FIG. 10A is a schematic plan view of front-band-member continuous bodies 31a immediately before being transported to a front-elastic-string cutting step S80 position, and FIG. 10B is a schematic plan view of the front-band-member continuous bodies 31a during and after passing through the step S80.
FIG. 11 is a schematic side view of an air-hole forming device 50, in which portions of the configurations are shown in a longitudinal cross-sectional view.
FIG. 12 is a schematic enlarged view taken along arrows XII-XII in FIG. 11.
FIG. 13 is a schematic side view of a producing step S200 for producing a first nonwoven fabric sheet 30mf1.
FIG. 14A is a schematic cross-sectional view of a state in which a pin member 55p is pressed into the sheet-like member 30mf.
FIG. 14B is a schematic cross-sectional view showing the pulling out of the pin member 55p that is pressed into the sheet-like member 30mf.
FIG. 15 is a diagram illustrating an arrangement pattern of pin members 55p on the outer circumferential surface of an intermediate roller 55.
FIG. 16 is a diagram illustrating an arrangement pattern of hole portions 51h in the outer circumferential surface of an upstream roller 51.
In FIG. 17, FIG. 17A is an enlarged view of a portion XVIIa in FIG. 15, and FIG. 17B is a view taken along arrows B-B in FIG. 17A.
In FIG. 18, FIG. 18A is a schematic side view of a front-side cutter device 80, and FIG. 18B is a view taken along arrows B-B in FIG. 18A.
FIG. 19A is a schematic side view of a step S210 performed at a location that is upstream of the air-hole forming step S50 position in the manufacturing line.
FIG. 19B is a schematic side view of the step S210 performed at a location that is upstream of the air-hole forming step S50 position in the manufacturing line.

### [Description of Embodiments]

At least the following matters will become clear with the description of this specification and the attached drawings.

A method for manufacturing a sheet-like member for an absorbent article,
the sheet-like member being formed from a composite sheet that is continuous in a predetermined direction,
the sheet-like member being provided with a plurality of through holes,
the method including:
   a transporting step of transporting the composite sheet in a direction of transport that is the predetermined direction,
      the composite sheet being constituted by a first nonwoven fabric sheet, a second nonwoven fabric sheet, and a plurality of elastic members,
      the first nonwoven fabric sheet being continuous in the predetermined direction,
      the second nonwoven fabric sheet being continuous in the predetermined direction and arranged overlapped with the first nonwoven fabric sheet in a thickness direction of the composite sheet,
      the plurality of elastic members being stretched in the predetermined direction and extending along the predetermined direction,
      the plurality of elastic members being inserted between and fixed to the first nonwoven fabric sheet and the second nonwoven fabric sheet in a side-by-side arrangement in a CD direction that intersects the predetermined direction; and
   a through-hole forming step of forming the through holes by pressing a press-in member in the thickness direction at positions between a pair of elastic members that are adjacent in the CD direction in the composite sheet,
      the through holes penetrating the composite sheet in the thickness direction,
   in the through-hole forming step,
      the press-in member being pressed in the thickness direction into a non-facing surface of the first nonwoven fabric sheet, and thereafter the press-in member being pulled out toward the non-facing surface,
      the non-facing surface being one of two surfaces of the first nonwoven fabric sheet,
      the non-facing surface not facing the second nonwoven fabric sheet,
      the non-facing surface of the first nonwoven fabric sheet being a surface that comes into contact with a support surface of a support member when constituent fibers of the first nonwoven fabric sheet are suctioned to and deposited on the support surface in order to produce the first nonwoven fabric sheet.

According to this method for manufacturing a sheet-like member for an absorbent article, it is possible to reduce the size of burrs that can project outward in the thickness direction from the non-facing surface of the first nonwoven fabric sheet when the through holes are formed. This will be described in detail below. First, when the press-in member is pressed, fibers of the first nonwoven fabric sheet cling to the press-in member and move to the press-in side in the thickness direction, and also, when the press-in member is pulled out, the fibers clinging to the press-in member move together therewith toward the non-facing surface, that is to say in the pull-out direction. For this reason, these fibers, which clung to the press-in member when being pulled out, project outward from the non-facing surface in the thickness direction, thus forming burrs on the non-facing surface. The ease of this movement of fibers in the thickness direction changes according to the density of the fibers. Specifically, if the fiber distribution density (g/cm³), which is the density of fibers, is low, the fibers easily move in the thickness direction, which is the direction of press-in the press-in member, but if the distribution density is high, the fibers do not easily move in the thickness direction, or in other words, the fibers easily move in a planar direction that is orthogonal to the thickness direction. In the step for producing the first nonwoven fabric sheet, when fibers are suctioned toward and deposited on the support surface of the support member in order to produce the first nonwoven fabric sheet, at a position close to the support surface, the suction force is more effective and the fibers are more easily arranged densely. This density decreases as the position moves away from the support surface in the depositing direction. In view of this, in the above-described manufacturing method, the non-facing surface of the first nonwoven fabric sheet is the surface that does not come into contact with the support surface. For this reason, the fiber distribution density is higher in a portion on this non-facing surface side than in a portion on the facing surface side, which is the opposite side. Accordingly, when the press-in member that was pressed into the first nonwoven fabric sheet is pulled out, the fibers in the portion on the facing surface side easily move together with the press-in member in the pull-out direction. But, the fibers that have a high distribution density in the portion on the non-facing surface side are located on the pull-out side of the portion on the facing surface side. For this reason, the fibers that are in the portion on the non-facing surface side, and which do not easily move in the thickness direction, prevent the fibers in the portion on the facing surface side from moving in the pull-out direction when they attempt to move together with the press-in member in the thickness direction, thus making it possible to suppress the projection of burrs from the non-facing surface.

In such a method for manufacturing a sheet-like member for an absorbent article, it is desirable
that a non-facing surface of the second nonwoven fabric sheet is one of two surfaces of the second nonwoven fabric sheet, and does not face the first nonwoven fabric sheet, and
that the non-facing surface of the second nonwoven fabric sheet is a surface that comes into contact with a support surface of a support member when constituent fibers of the second nonwoven fabric sheet are suctioned to and deposited on the support surface in order to produce the second nonwoven fabric sheet.

According to this method for manufacturing a sheet-like member for an absorbent article, it is possible to reduce the size of burrs that can project outward in the thickness direction from the non-facing surface of the second nonwoven fabric sheet when the through holes are formed. This will be described in detail below. The press-in member is first pressed into the first nonwoven fabric sheet, and then pressed into the second nonwoven fabric sheet. When being pressed into the second nonwoven fabric sheet, the press-in member is pressed first into the portion on the facing surface side that faces the first nonwoven fabric sheet, and then into the portion on the non-facing surface side that does not face the first nonwoven fabric sheet. In other words, it is pressed into the portion on the non-facing surface side last. When fibers clinging to the press-in members at this time move together therewith in the direction of press-in, burrs are formed in a manner of projecting outward from the non-facing surface. However, the non-facing surface of the second nonwoven fabric sheet is the surface that came into contact with the support surface of the support member when the second nonwoven fabric sheet was produced, and as described above, the fiber distribution density is high in this portion on the non-facing surface side. For this reason, the fibers in the portion on the non-facing surface side do not easily move in the thickness direction, which is the direction of press-in, and as a result, it is possible to suppress the projection of burrs from the non-facing surface of the second nonwoven fabric sheet.

In such a method for manufacturing a sheet-like member for an absorbent article, it is desirable that
that the composite sheet has a fixing portion in which the first nonwoven fabric sheet and the second nonwoven fabric sheet are fixed to each other, and
that the through holes are formed in the composite sheet such that the through holes are not overlapped with the fixing portion.

According to this method for manufacturing a sheet-like member for an absorbent article, it is possible to effectively prevent a problem that can occur if the through holes are formed so as to be overlapped with the fixing portion, such as the problem of difficulty in punching the through holes due to an increase in punching resistance when punching the through holes, which is caused by the fixing portion, and the problem where the softness of the sheet-like member is impaired by hardening of the burrs caused by the fixing portion.

In such a method for manufacturing a sheet-like member for an absorbent article, it is desirable
that the composite sheet has a direct fixing portion in which the first nonwoven fabric sheet and the second nonwoven fabric sheet are fixed to each other without using the elastic members, and
that the through holes are formed in the composite sheet such that the through holes are not overlapped with the direct fixing portion.

According to this method for manufacturing a sheet-like member for an absorbent article, it is possible to effectively prevent a problem that can occur if the through holes are formed so as to be overlapped with the direct fixing portion, such as the problem of difficulty in punching the through holes due to an increase in punching resistance when punching the through holes, which is caused by the direct fixing portion, and the problem where the softness of the sheet-like member is impaired by hardening of the burrs caused by the direct fixing portion.

In such a method for manufacturing a sheet-like member for an absorbent article, it is desirable that
when a surface that is one of two surfaces of the first nonwoven fabric sheet and that is not in contact with the support surface of the support member is defined as a non-contacting surface,
a driven roller is arranged at a position that is upstream, with respect to the direction of transport, of a through hole formation position at which the through holes are formed in the composite sheet in the through-hole forming step, and
the driven roller comes into contact with the non-contacting surface of the first nonwoven fabric sheet being transported, and is thus driven to rotate by rotation force received from the first nonwoven fabric sheet.

According to this method for manufacturing a sheet-like member for an absorbent article, the fibers in the portion on the non-contacting surface side of the first nonwoven fabric sheet effectively become relaxed due to shearing force that is applied through contact with the driven roller. This therefore makes it possible to lower the punching resistance when forming the through holes in the first nonwoven fabric sheet, thus making it easier to form the through holes.

In such a method for manufacturing a sheet-like member for an absorbent article, it is desirable
that the driven roller is a dancer roller that is guided to be capable of moving in the predetermined direction in which the first nonwoven fabric sheet is continuous,
that a loop is formed in the first nonwoven fabric sheet by the first nonwoven fabric sheet being wound on the dancer roller, and
that a predetermined load is applied to the dancer roller in a direction according to which the loop increases in size.

According to this method for manufacturing a sheet-like member for an absorbent article, the above-described load is applied to the dancer roller. Accordingly, due to this contact with the dancer roller, it is possible to increase the amount of shearing force that is applied to the first nonwoven fabric sheet. This therefore makes it possible to more effectively relax the fibers in the portion on the non-contacting surface side of the first nonwoven fabric sheet with use of this shearing force.

In such a method for manufacturing a sheet-like member for an absorbent article, it is desirable that
the non-facing surface forms a skin-side surface in the absorbent article.

According to this method for manufacturing a sheet-like member for an absorbent article, the non-facing surface with reduced-size burrs forms the skin-side surface. This therefore enables providing a favorable texture when touched from the skin side, thus making it possible to manufacture an absorbent article that has a favorable texture.

In such a method for manufacturing a sheet-like member for an absorbent article, it is desirable that
the non-facing surface forms a non-skin-side surface in the absorbent article.

According to this method for manufacturing a sheet-like member for an absorbent article, the non-facing surface with reduced-size burrs forms the non-skin-side surface. This therefore enables giving the through holes a nice appearance when viewed from the non-skin side, thus making it possible to manufacture an absorbent article that has a favorable appearance.

A device for manufacturing a sheet-like member for an absorbent article,
the sheet-like member being formed from a composite sheet that is continuous in a predetermined direction,
the sheet-like member being provided with a plurality of through holes,
the device including:
   a transporting device that transports the composite sheet in a direction of transport that is the predetermined direction,
      the composite sheet being constituted by a first nonwoven fabric sheet, a second nonwoven fabric sheet, and a plurality of elastic members,
      the first nonwoven fabric sheet being continuous in the predetermined direction,
      the second nonwoven fabric sheet being continuous in the predetermined direction and arranged overlapped with the first nonwoven fabric sheet in a thickness direction of the composite sheet,
      the plurality of elastic members being stretched in the predetermined direction and extending along the predetermined direction,
      the plurality of elastic members being inserted between and fixed to the first nonwoven fabric sheet and the second nonwoven fabric sheet in a side-by-side arrangement in a CD direction that intersects the predetermined direction; and
   a through-hole forming device that forms the through holes by pressing a press-in member in the thickness direction at positions between a pair of elastic members that are adjacent in the CD direction in the composite sheet,
      the through holes penetrating the composite sheet in the thickness direction,
   in the through-hole forming device,
      the press-in member being pressed in the thickness direction into a non-facing surface of the first nonwoven fabric sheet, and thereafter the press-in member being pulled out toward the non-facing surface,
      the non-facing surface being one of two surfaces of the first nonwoven fabric sheet,
      the non-facing surface not facing the second nonwoven fabric sheet,
      the non-facing surface of the first nonwoven fabric sheet being a surface that comes into contact with a support surface of a support member when constituent fibers of the first nonwoven fabric sheet are suctioned to and deposited on the support surface in order to produce the first nonwoven fabric sheet.

According to this device for manufacturing a sheet-like member related to an absorbent article, it is possible to achieve actions and effects similar to those of the manufacturing method described above.

### Embodiments

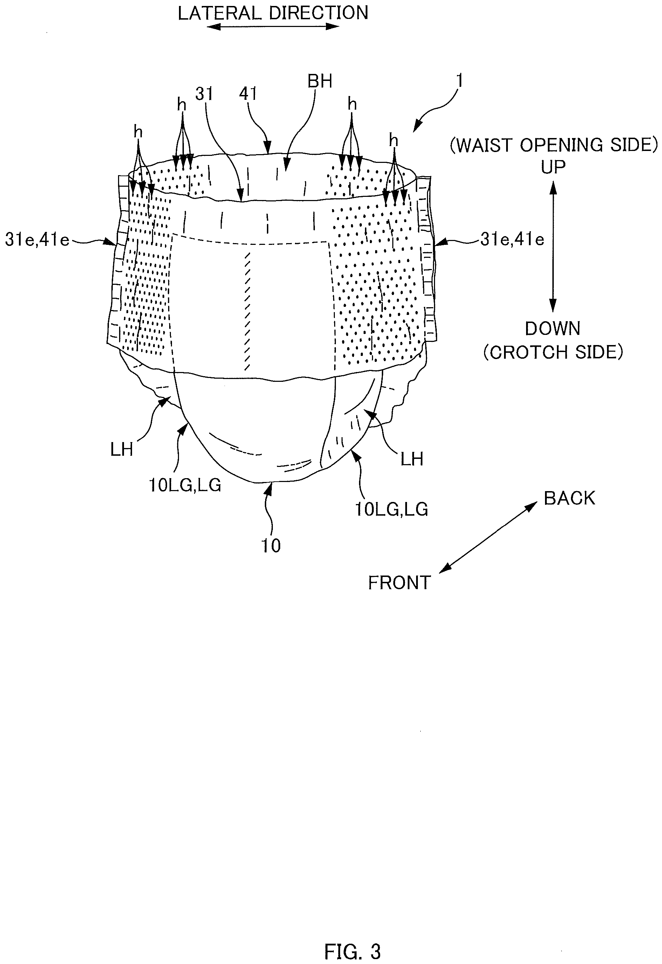

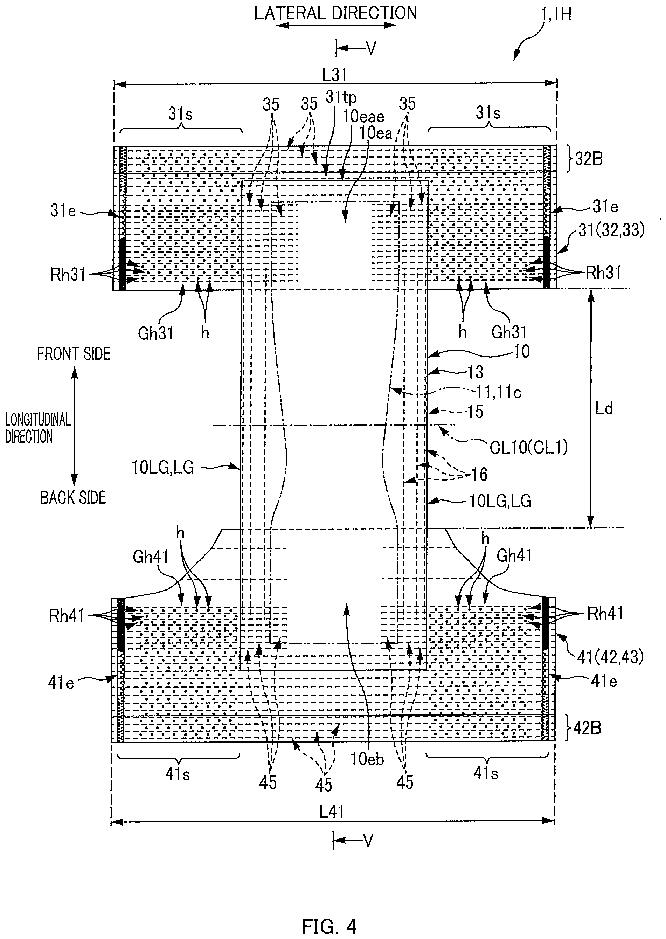

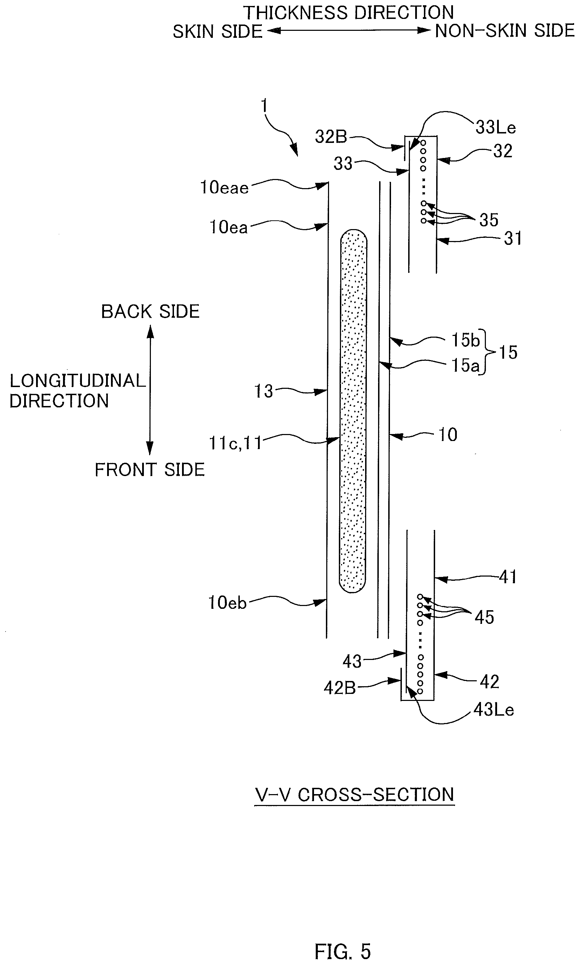

A method and device according to embodiments of the present invention for manufacturing a sheet-like member for an absorbent article are used in a manufacturing line for manufacturing a disposable diaper 1, which is one example of the absorbent article. FIG. 3 is a schematic perspective view of a pants-shaped state of a three-piece type of diaper 1, which is one example of the disposable diaper 1, as viewed from a front side. Also, FIG. 4 is a schematic plan view of the diaper 1 in an unfolded state, as viewed from the skin side, and FIG. 5 is a cross-sectional view taken along V-V in FIG. 4.

In the pants-shaped state shown in FIG. 3, the diaper 1 has an up-down direction, a lateral direction, and a front-back direction as three directions that are orthogonal to each other. In the following, the upper side and the lower side in the up-down direction in the pants-shaped state will also be respectively called the "waist opening side" and the "crotch side", and the front side and the back side in the front-back direction will also be respectively called the "front side" and the "back side". Also, in the unfolded state shown in FIGS. 4 and 5, the diaper 1 has a longitudinal direction, a lateral direction, and a thickness direction as three directions that are orthogonal to each other. In the following, one side and the other side in the longitudinal direction in the unfolded state will also be respectively called the "front side" and the "back side", and one side and the other side in the thickness direction will also be respectively called the "skin side" and the "non-skin side". Note that the lateral direction has the same meaning in the pants-shaped state and the unfolded state. Also, the longitudinal direction in the unfolded state conforms to the up-down direction in the pants-shaped state, and the thickness direction in the unfolded state conforms to the front-back direction in the pants-shaped state. Furthermore, in the unfolded state shown in FIGS. 4 and 5, the diaper 1 is shown in a state of being stretched out to the extent that no contractive force whatsoever is exerted by elastic strings 16, 35, and 45 that serve as later-described elastic members for giving stretchability to the diaper 1.

Because the diaper 1 is a so-called three piece type of diaper, in the unfolded state shown in FIG. 4, it has an absorbent main body 10 for absorbing excrement as a first component, a front band member 31 as a second component, and a back band member 41 as a third component. Specifically, in the state where the front band member 31 and the back band member 41 are arranged parallel to each other with a gap therebetween in the longitudinal direction, the absorbent main body 10 spans between the members 31 and 41, and end portions 10ea and 10eb in the longitudinal direction of the absorbent main body 10 are respectively joined and fixed to the closest ones out of the band members 31 and 41, thus having an approximately H-shaped appearance in a plan view.

In this approximately H-shaped unfolded state, the absorbent main body 10 is folded in two at a folding position that is a predetermined position CL10 (position corresponding to a central position CL1 of the diaper 1 in the longitudinal direction) in the longitudinal direction of the absorbent main body 10, and when the band members 31 and 41, which face each other in this folded state, are joined by welding in lateral end portions 31e and 41e, the band members 31 and 41 join together to form a ring shape, thus obtaining the diaper 1 in the pants-shaped state in which a waist opening BH and a pair of leg openings LH are formed as shown in FIG. 3.

Note that in the unfolded state shown in FIGS. 4 and 5, the absorbent main body 10 is approximately rectangular in a plan view. The lengthwise direction of the absorbent main body 10 conforms to the longitudinal direction. Also, the absorbent main body 10 includes an absorbent body 11, a top sheet 13 that covers the absorbent body 11 from the skin side, and a back sheet 15 that covers the absorbent body 11 from the non-skin side.

The absorbent body 11 has a liquid-absorbent absorbent core 11c, and a core-wrapping sheet that is made of tissue paper or the like (not shown) and covers the outer circumferential surface of the core 11c. The absorbent core 11c is a molded body that is made of a predetermined liquid absorbent material and is approximately hourglass-shaped in a plan view, as one example of a predetermined shape. Examples of the liquid absorbent material include liquid-absorbent fibers such as pulp fibers, and liquid-absorbent particles made of a superabsorbent polymer (so-called SAP) or the like.

The top sheet 13 is a liquid-permeable sheet that is made of nonwoven fabric and has a planar size according to which it projects from the absorbent body 11 in the longitudinal direction and the lateral direction. The back sheet 15 is also a sheet having a planar size according to which it projects from the absorbent body 11 in the longitudinal direction and the lateral direction, and one example is the double-layer structure laminate sheet 15 shown in FIG. 5. Specifically, this laminate sheet 15 has a liquid-impermeable leak-proof sheet 15a made of a polyethylene film (PE) or a polypropylene film (PP) on the skin side, and has an exterior sheet 15b made of nonwoven fabric on the non-skin side.

When the absorbent body 11 is sandwiched between the top sheet 13 and the back sheet 15, the portions of the sheets 13 and 15 that project from the absorbent body 11 in the longitudinal direction and the lateral direction are joined together by adhesion, welding, or the like to obtain a frame-like shape, thus forming the absorbent main body 10. Note that the exterior sheet 15b may be omitted, and the back sheet 15 may have only the leak-proof sheet 15a.

Also, as shown in this example in FIG. 4, portions 10LG of the absorbent main body 10 that are laterally outward of the absorbent body 11 may be provided with leg gathers LG that stretch and contract in the longitudinal direction. These leg gathers LG constitute portions of the leg openings LH. Also, the leg gathers LG are given stretchability by fixing elastic strings 16, which are elastic members that extend in the longitudinal direction, to the portions 10LG in a state of being stretched in the longitudinal direction. Note that in addition to these leg gathers LG, in order to prevent lateral leakage, so-called barrier cuffs (not shown) may be provided as leak-proof wall portions on the two lateral sides of the absorbent main body 10.

Also, as shown in FIG. 4, the front band member 31 is a sheet member that has an approximately rectangular shape in a plan view, and is made up of nonwoven fabric sheets 32 and 33. In this example, as shown in FIG. 5, the front band member 31 is formed by joining together the two overlapped nonwoven fabric sheets 32 and 33 with use of hot-melt adhesive that has been applied to later-described elastic strings 35. Also, as shown in FIGS. 4 and 5, the laterally central portion of the front band member 31 is overlaid on, from the non-skin side, and joined to a front longitudinal end portion 10ea of the absorbent main body 10.

Similarly to the front band member 31, the back band member 41 is also a sheet member that has an approximately rectangular shape in a plan view and is made up of nonwoven fabric sheets 42 and 43, and in this example, as shown in FIG. 5, the back band member 41 is formed by joining together the two overlapped nonwoven fabric sheets 42 and 43 with use of a hot-melt adhesive that has been applied to later-described elastic strings 45. Also, as shown in FIGS. 4 and 5, the laterally central portion of the back band member 41 is overlaid on, from the non-skin side, and joined to a back longitudinal end portion 10eb of the absorbent main body 10.

Note that the content described below is the same for both the front band member 31 and the back band member 41. For this reason, the following describes only the front band member 31 as a representative for both, and corresponding portions of the back band member 41 are simply indicated in parentheses.

As shown in FIGS. 4 and 5, multiple elastic strings 35, 35... (45, 45...), which are elastic members that extend in the lateral direction, are inserted side-by-side in the longitudinal direction between the two nonwoven fabric sheets 32 and 33 (42 and 43) of the front band member 31 (41), and are joined and fixed to the nonwoven fabric sheets 32 and 33 (42 and 43) with use of a hot-melt adhesive, in a state of being stretched in the lateral direction. Accordingly, the front band member 31 (41) is given stretchability in the lateral direction.

Also, in this example, as shown in FIG. 4, the elastic strings 35 (45) are non-continuous in at least a portion (e.g., the laterally central portion) of the front band member 31 (41) that is overlapped with the absorbent core 11c, in order to prevent the formation of wrinkles in the absorbent body 11 for example. Accordingly, stretchability is not given to this portion.

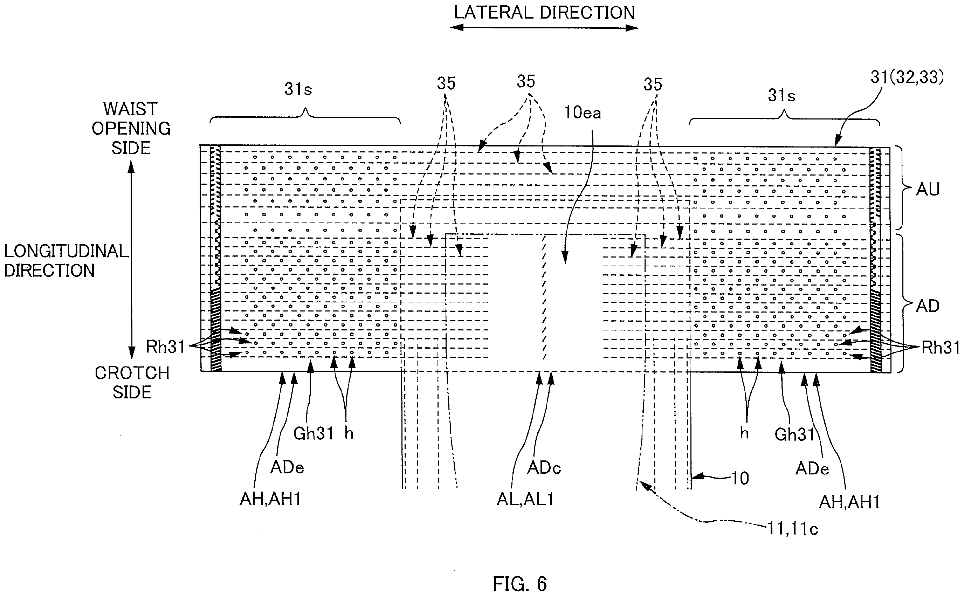

Specifically, as shown in the schematic plan view of the front band member 31 as viewed from the non-skin side in the thickness direction in FIG. 6, if the front band member 31 (41) is divided into two regions AU and AD in the longitudinal direction, with "upper region AU" referring to the region AU located on the waist opening BH side in the longitudinal direction, and "lower region AD" referring to the region AD located on the crotch side, then the absorbent core 11c of the absorbent main body 10 is substantially not overlapped with the upper region AU. For this reason, the upper region AU is given stretchability over substantially the entire length in the lateral direction. In other words, the elastic strings 35, 35... (45, 45...) are provided extending over substantially the entire length in the lateral direction in the upper region AU.

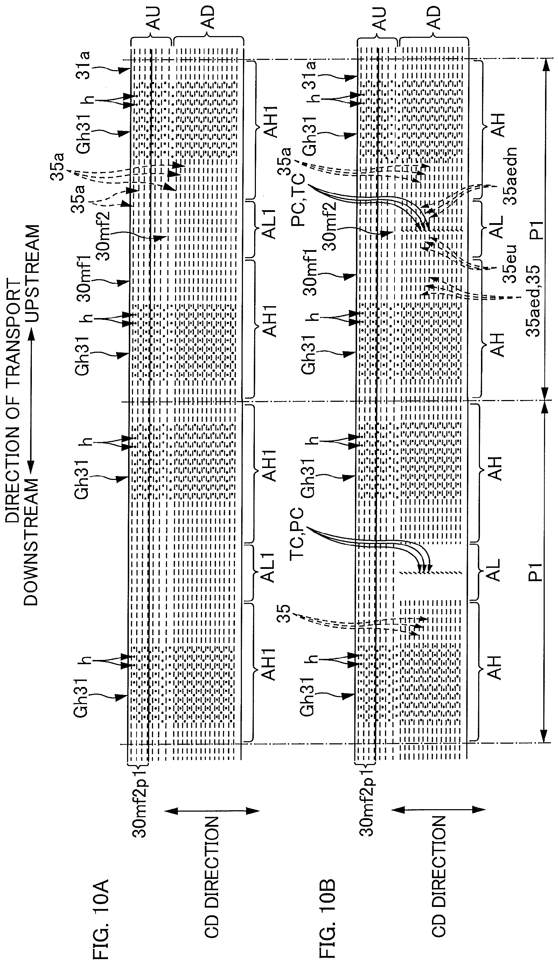

Meanwhile, in the lower region AD, the absorbent core 11c of the absorbent main body 10 is overlapped with a laterally central region ADc, and therefore the elastic strings 35, 35... are not arranged in the central portion of this central region ADc, thus forming a non-stretchy region AL having substantially no lateral stretchability in this central portion of the central region ADc. It should be noted that the absorbent core 11c is substantially not overlapped with two end regions ADe located on respective sides of the central region ADc, and therefore the elastic strings 35, 35... (45, 45...) are arranged in these end regions Ade, thus forming two stretchy regions AH that have higher stretchability than the non-stretchy region AL, in the two end regions ADe. Note that the non-stretchy region AL and the stretchy regions AH are formed in the lower region AD by cutting continuous bodies 35a (45a) of the elastic strings (see FIGS. 10A and 10B), which are arranged traversing the central portion in the lateral direction, the cutting being made in the central portion. This cutting will be described later.

Also, the fiber density of these elastic strings 35 (45) is 400 dtex to 1000 dtex for example, and one specific example of the elastic strings 35 (45) is LYCRA (registered trademark) . Note that there is no limitation whatsoever to this.

Furthermore, in this example, as shown in FIG. 5, the planar size of the nonwoven fabric sheet 32 (42), which is the one of the two nonwoven fabric sheets 32 and 33 (42 and 43) that is located on the non-skin side in the thickness direction, is a size according to which it projects longitudinally outward from the nonwoven fabric sheet 33 (43) that is located on the skin side. Also, the projecting portion of the nonwoven fabric sheet 32 (42) is folded back inward in the longitudinal direction, and this folded portion 32B (42B) covers a longitudinal end portion 33Le (43Le) of the nonwoven fabric sheet 33 (43) from the skin side, but there is no limitation whatsoever to this.

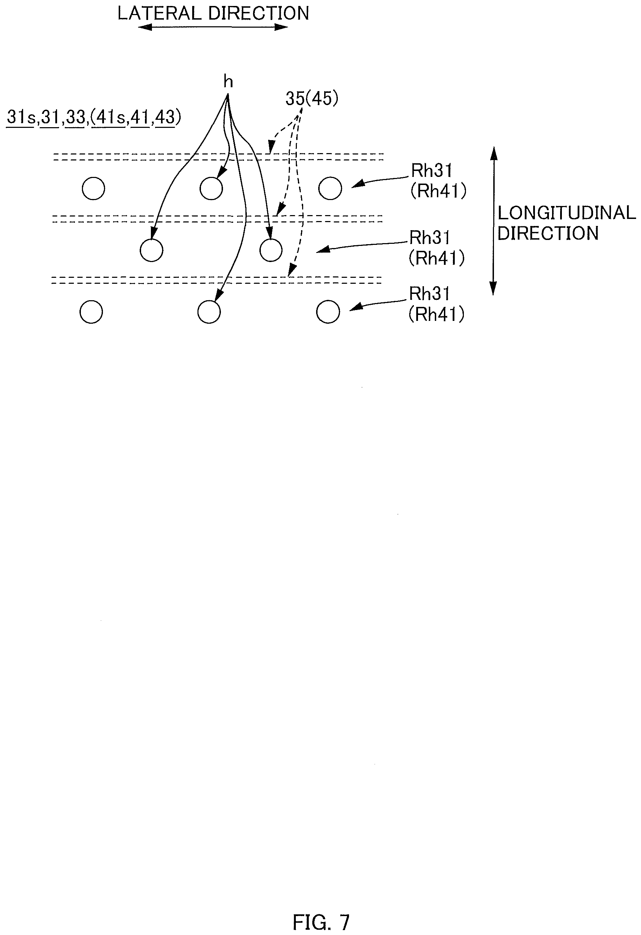

Also, as shown in FIGS. 4 and 6, in each of the two lateral sides of the front band member 31 (41), there is a portion 31s (41s) (hereinafter, also called a no-absorbent-body portion 31s (41s)) that includes the end region ADe and is not overlapped by the absorbent main body 10. In order to improve breathability in this no-absorbent-body portion 31s (41s), multiple air holes h (h) that pass through the no-absorbent-body portion 31s (41s) are formed discretely in a predetermined arrangement pattern, and these air holes h (h) form an air hole group Gh31 (Gh41), which is a group of air holes h. Specifically, in this example, as shown in the schematic enlarged view in FIG. 7, in each of the no-absorbent-body portions 31s (41s), multiple air holes h, h... (h, h...) are formed side-by-side with a predetermined formation pitch at positions between pairs of elastic strings 35 (45) that are adjacent in the longitudinal direction, and therefore these air holes h, h... (h, h...) form air hole rows Rh31 (Rh41) that extend in the lateral direction. Also, pairs of air hole rows Rh31 (Rh41) that are adjacent in the longitudinal direction are shifted from each other in the lateral direction by half the formation pitch. Accordingly, the air holes h, h... (h, h...) form the air hole group Gh31 (Gh41) that has a substantially staggered arrangement as shown in FIGS. 4 and 6. Note that there is no limitation whatsoever to this. For example, the air holes h, h... (h, h...) may be formed in a lattice arrangement.

Furthermore, in this example, the air holes h, h... are each formed with a target opening shape, which is a perfect circle having a diameter of 0.2 mm to 3 mm, but there are also air holes h, h... that are not a perfect circle due to formation precision problems. The diameter of such non-perfect circle air holes h varies according to the position on the circumference of the air hole h, but even these varying diameters fall in the range of 0.2 mm to 3 mm, for example. Accordingly, the air holes h can quickly exhibit anticipated breathability. It should be noted that the target opening shape of the air holes h is not limited in any way to the aforementioned perfect circle. For example, the target opening shape may be any polygon, such as an equilateral triangle or a square.

Also, in this example, the two nonwoven fabric sheets 32 and 33 for the front band member 31 and the two nonwoven fabric sheets 42 and 43 for the back band member 41 are each made of spunbond nonwoven fabric. It should be noted that there is no limitation whatsoever to this, and it is possible to use a different type of nonwoven fabric such as SMS (spunbond/meltblown/spunbond) nonwoven fabric. Also, standalone fibers made of polypropylene (PP), which is a representative example of a thermoplastic resin, are used as the fibers that constitute the nonwoven fabric, but there is no limitation whatsoever to this. For example, standalone fibers made of another type of thermoplastic resin, such as polyethylene (PE), may be used, and furthermore, it is possible to use composite fibers that have a core-sheath structure including PE and PP, for example.

This diaper 1 is manufactured in a manufacturing line. In this manufacturing line, an intermediate product 1m of the diaper 1 is transported along a predetermined direction of transport (corresponding to a transporting step). During this transporting, various types of processing are performed on the intermediate product 1m, whereby each time processing is performed, the shape of the intermediate product 1m successively changes, and ultimately the diaper 1 shown in FIG. 3 is completed.

Note that in the following, the width direction of the manufacturing line is also called the "CD direction". Moreover, in this example, the CD direction conforms to the horizontal direction. In this manufacturing line, the direction of transport along which the intermediate product 1m is transported is any direction in a plane that is orthogonal to the CD direction. In other words, the direction of transport is a direction defined by both the vertical up-down direction and the horizontal front-back direction. Note that the terms "up-down direction" and "front-back direction" used here are directions that are different from and not directly related to the terms "up-down direction" and the "front-back direction" used in the above description of the diaper 1.

Also, the intermediate product 1m is transported by appropriate transporting devices such as a belt transporter and transporting rollers. Accordingly, unless particularly stated otherwise, the intermediate product 1m is assumed to be transported in the direction of transport by these transporting devices. Note that examples of belt transporters include a normal belt transporter that has a circulating endless belt as a transporting surface, and a suction belt transporter that includes an endless belt whose outer circumferential surface has a suction function.

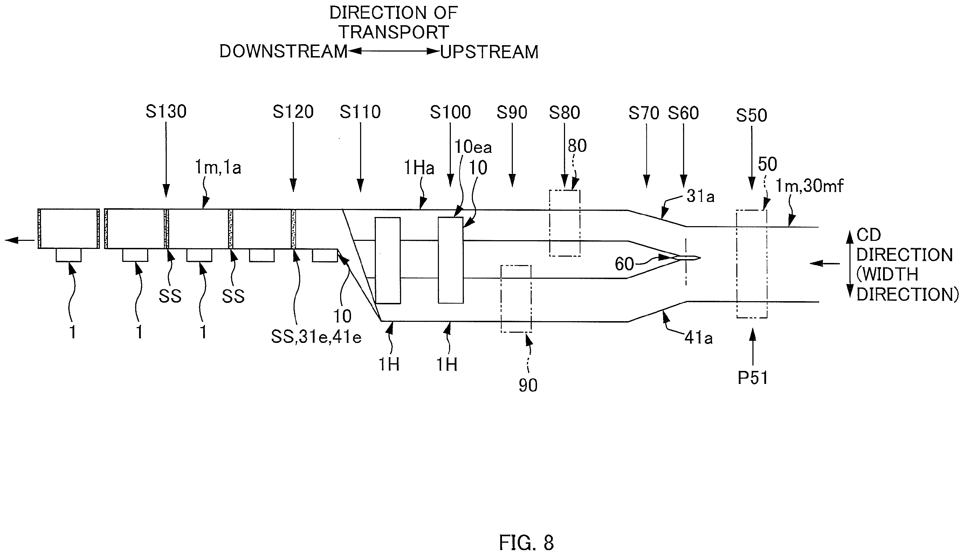

FIG. 8 is a schematic plan view of the manufacturing line for illustrating various types of processing performed in the manufacturing line. The manufacturing line has an air-hole forming step S50 (corresponding to a through-hole forming step), a slitting step S60, a gap forming step S70, a front-elastic-string cutting step S80, a back-elastic-string cutting step S90, an absorbent-main-body attaching step S100, a folding step S110, a side sealing step S120, and a cutting step S130. The positions at which these steps S50 to S130 are performed are arranged side-by-side in the stated order from upstream to downstream in the direction of transport.

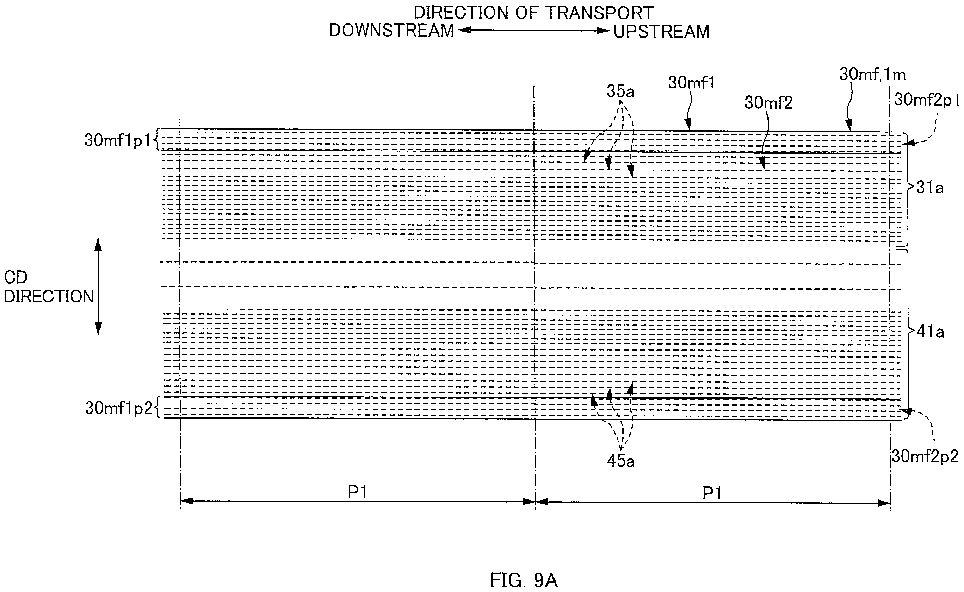

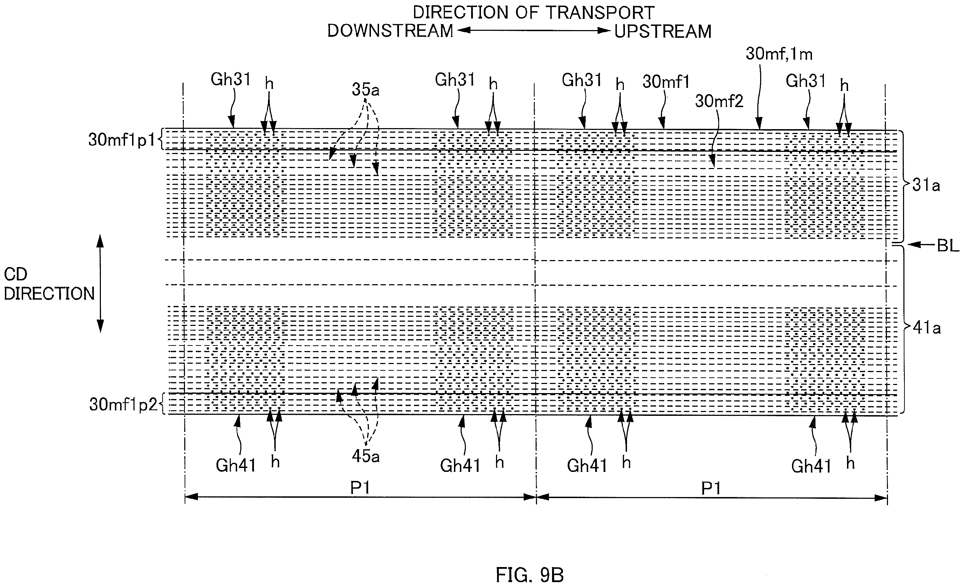

FIG. 9A is a schematic plan view of the intermediate product 1m immediately before being transported to the air-hole forming step S50 position, and FIG. 9B is a schematic plan view of the intermediate product 1m immediately after passing through the air-hole forming step S50.

As can be understood from a comparison of FIGS. 9A and 9B, in this air-hole forming step S50, the previously mentioned air holes h, h... (corresponding to through holes) are formed in the intermediate product 1m, which is the base material for both the front band member 31 and the back band member 41. Here, in this manufacturing line, the intermediate product 1m is transported using so-called side flow transporting. Specifically, the intermediate product 1m is transported in an orientation in which the lateral direction of the diaper 1 conforms to the direction of transport, and the longitudinal direction conforms to the CD direction. For this reason, as show in FIG. 9A, when sent from an upstream step to the air-hole forming step S50 position, the intermediate product 1m is substantially constituted by one sheet-like member 30mf, in which a front-band-member continuous body 31a, which has multiple front band members 31 that are connected in the lateral direction, and a back-band-member continuous body 41a, which has multiple back band members 41 that are connected in the lateral direction, are connected in the CD direction as a single body.

More specifically, this sheet-like member 30mf has a nonwoven fabric sheet 30mf1 (corresponding to a first nonwoven fabric sheet, and hereinafter also called the first nonwoven fabric sheet 30mf1) and a nonwoven fabric sheet 30mf2 (corresponding to a second nonwoven fabric sheet, and hereinafter also called the second nonwoven fabric sheet 30mf2): the nonwoven fabric sheet 30mf1 is continuous in the direction of transport and has a size in the CD direction that corresponds to the sum value of the longitudinal dimension of the nonwoven fabric sheet 32 of the front band member 31 and the longitudinal dimension of the nonwoven fabric sheet 42 of the back band member 41; and the nonwoven fabric sheet 30mf2 is continuous in the direction of transport and has a size in the CD direction that corresponds to the sum value of the longitudinal dimension of the nonwoven fabric sheet 33 of the front band member 31 and the longitudinal dimension of the nonwoven fabric sheet 43 of the back band member 41. The first nonwoven fabric sheet 30mf1 and the second nonwoven fabric sheet 30mf2 are overlaid on each other so as to be overlapped in the thickness direction. Also, elastic-string continuous bodies 35a and 45a, which are to form the previously mentioned elastic strings 35 and 45, are inserted between the first nonwoven fabric sheet 30mf1 and the second nonwoven fabric sheet 30mf2 side-by-side in the CD direction in a state of being stretched in the direction of transport and continuous in the direction of transport. In this inserted state, the elastic-string continuous bodies 35a and 45a are fixed to the first nonwoven fabric sheet 30mf1 and the second nonwoven fabric sheet 30mf2 with use of a hot-melt adhesive applied to the continuous bodies 35a and 45a, and the first nonwoven fabric sheet 30mf1 and the second nonwoven fabric sheet 30mf2 are indirectly fixed to each other via the elastic-string continuous bodies 35a and 45a. Furthermore, the first nonwoven fabric sheet 30mf1 has a larger CD direction size than the second nonwoven fabric sheet 30mf2, and therefore the first nonwoven fabric sheet 30mf1 has portions 30mf1p1 and 30mflp2 that project from the second nonwoven fabric sheet 30mf2 on the two sides in the CD direction. These projecting portions 30mf1p1 and 30mflp2 are folded inward in the CD direction, thus forming portions that correspond to the previously mentioned folded portions 32B and 42B.

Then, in the air-hole forming step S50, a pair of air hole groups Gh31 pertaining to the front band member 31 are repeatedly formed in the intermediate product 1m shown in FIG. 9A, which is the sheet-like member 30mf (corresponding to a composite sheet), in a region thereof on one side in the CD direction as shown in FIG. 9B at a product pitch P1 in the direction of transport. Also, a pair of air hole groups Gh41 pertaining to the back band member 41 are formed in a region on the other side at the product pitch P1 in the direction of transport. Note that the product pitch P1 mentioned here is substantially the same value as overall lateral lengths L31 and L41 of the front band member 31 and the back band member 41 in the unfolded state shown in FIG. 4.

Next, in the slitting step S60, the sheet-like member 30mf is folded in two in the CD direction as shown in FIG. 8, thus forming the front-band-member continuous body 31a and the back-band-member continuous body 41; the front-band-member continuous body 31a includes the elastic-string continuous bodies 35a in the uncut state, and the back-band-member continuous body 41a includes the elastic-string continuous bodies 45a in the uncut state.

In the subsequent gap forming step S70, the front-band-member continuous body 31a and the back-band-member continuous body 41a, which form the intermediate product 1m, are each moved outward in the CD direction. Accordingly, a gap is formed in the CD direction between the continuous bodies 31a and 41a, and the size of this gap corresponds to a longitudinal gap Ld between the front band member 31 and the back band member 41 in the diaper 1 in the unfolded state shown in FIG. 4.

In the subsequent front-elastic-string cutting step S80, as the front-band-member continuous body 31a passes, the elastic-string continuous bodies 35a are cut in a region AL1 that corresponds to the non-stretchy region AL in the continuous body 31a, thus forming the non-stretchy region AL in the front-band-member continuous body 31a. This will be described in detail below.

First, in this front-band-member continuous body 31a, as shown in the schematic plan view in FIG. 10A, the region on one side in the CD direction corresponds to the previously mentioned upper region AU in the front band member 31, and the region on the other side likewise corresponds to the lower region AD. At this point in time, in both of these regions, the elastic-string continuous bodies 35a extend in the direction of transport and are stretched in the direction of transport. It should be noted that in the region on the one side that corresponds to the upper region AU, the elastic-string continuous bodies 35a are fixed with a hot-melt adhesive over the entire length in the direction of transport, but in the region on the other side that corresponds to the lower region AD, there are fixed regions AH1, in which the elastic-string continuous bodies 35a are fixed, and a non-fixed region AL1, in which the elastic-string continuous bodies 35a are not fixed. The fixed regions and non-fixed region are side-by-side in the direction of transport. The non-fixed region AL1 is the same size as the previously mentioned non-stretchy region AL in the direction of transport, and is arranged at the product pitch P1 in the direction of transport.

Accordingly, as shown in the right portion in FIG. 10B, when the elastic-string continuous bodies 35a are cut at a predetermined position PC in the non-fixed region AL1, downstream end portions 35aed of the elastic-string continuous bodies 35a are cut away from the continuous bodies 35a. Accordingly, upstream portions 35eu of the cut-away elastic strings 35 contract toward the fixed region AH1 that is located on the downstream side. Also, due to this cutting, portions 35aedn of the elastic-string continuous bodies 35a, which become new downstream end portions thereof, contract toward the fixed region AH1 that is located on the upstream side. Accordingly, as shown in the left portion in FIG. 10B, the non-fixed region AL1 becomes a region in which the elastic strings 35 do not exist, and therefore the non-fixed region AL1 forms the previously mentioned non-stretchy region AL. On the other hand, as shown in FIG. 10B, in the fixed regions AH1, the cut-away elastic strings 35 and the newly-become-downstream end portions 35aedn remain due to being fixed with a hot-melt adhesive, and therefore these elastic strings 35 give stretchability to the fixed regions AH1. Accordingly, the fixed regions AH1 form the previously mentioned stretchy regions AH.

In the subsequent back-elastic-string cutting step S90, as the back-band-member continuous body 41a shown in FIG. 8 passes, the elastic-string continuous bodies 45a are cut in a region AL1 that corresponds to the non-stretchy region AL in the continuous body 41a, thus forming the non-stretchy region AL in the back-band-member continuous body 41a. Note that the processing for forming the non-stretchy region AL through this cutting is substantially the same as the above-described cutting performed on the front-band-member continuous body 31a. A description therefore will not be given for this.

In the subsequent absorbent-main-body attaching step S100, the cutform-shaped absorbent main body 10, which is produced using other steps that are not shown, is fixed in a manner of spanning between the two band member continuous bodies 31a and 41a that are sent as the intermediate product 1m from the elastic string cutting steps S80 and S90. Accordingly, the intermediate product 1m becomes an approximately ladder-shaped diaper continuous body 1Ha that is formed by a series of diapers 1H that are unfolded and approximately H-shaped as shown in FIG. 4.

In the subsequent folding step S110, as shown in FIG. 8, the approximately ladder-shaped diaper continuous body 1Ha, which is the intermediate product 1m, is folded in two such that the front-band-member continuous body 31a and the back-band-member continuous body 41a are overlaid on one another in the thickness direction.

In the subsequent side sealing step S120, the front-band-member continuous body 31a and the back-band-member continuous body 41a, which were overlaid on one another in the folding step S110, are welded at positions corresponding to the lateral end portions 31e and 41e of the diaper 1 to form side seal portions SS, thus being fixed in a state of being folded in two. As a result, the intermediate product 1m becomes the pull-on diaper continuous body 1a in which multiple pull-on diapers 1 are connected in the lateral direction.

Then, in the final cutting step S130, the pull-on diaper continuous body 1a, which is the intermediate product 1m, is cut at each of the side seal portions SS, and individual pull-on diapers 1 are thus manufactured.

The following describes the steps S50 to S100 in further detail, but the steps from the folding step S110 to step S130 will not be described any further because it is clear that they can be appropriately realized using a known method.

### Air-hole forming step S50

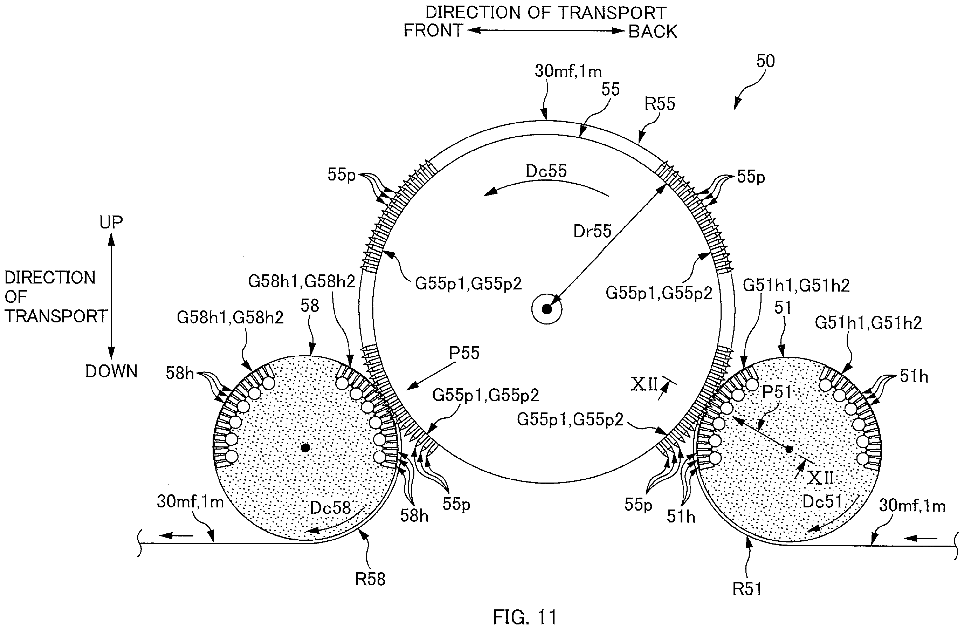

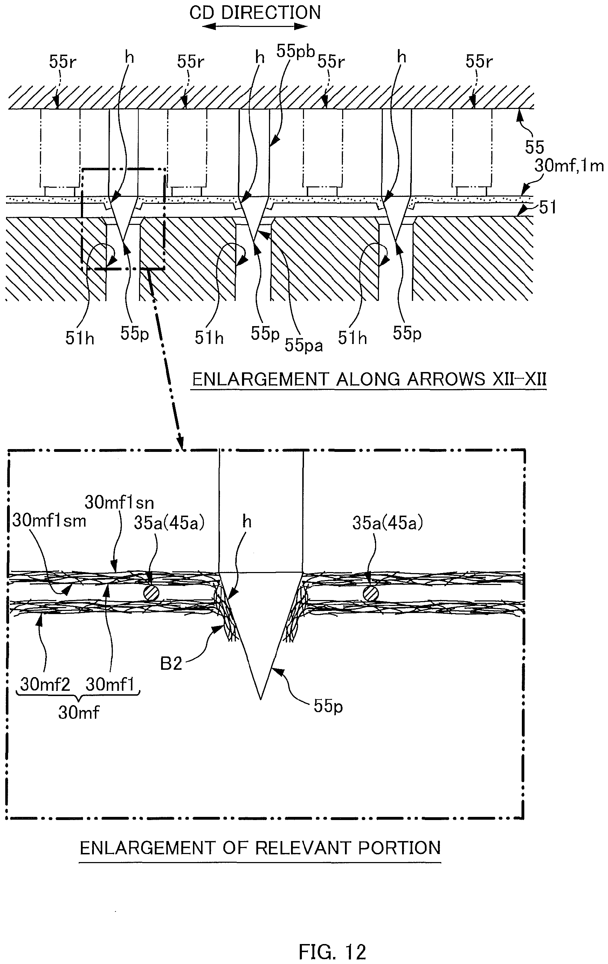

In the air-hole forming step S50, an air-hole forming device 50 (corresponding to a through-hole forming device) is provided for forming the air holes h in the sheet-like member 30mf. FIG. 11 is a schematic side view of the device 50, in which portions of the configuration (upstream roller 51 and downstream roller 58) are shown in a longitudinal cross-sectional view, and FIG. 12 is a schematic side view taken along arrows XII-XII in FIG. 11.

As shown in FIG. 11, the air-hole forming device 50 has three rollers 51, 55, and 58 that are driven to rotate about rotation shafts that extend in the CD direction. Specifically, the rollers 51, 55, and 58 are arranged side-by-side as an upstream roller 51, an intermediate roller 55, and a downstream roller 58 in this order from upstream to downstream in the direction of transport. Also, the intermediate roller 55 is arranged adjacent to the upstream roller 51 and the downstream roller 58 such that the outer circumferential surfaces thereof face each other. The above-described sheet-like member 30mf, which is sent as the intermediate product 1m to the air-hole forming device 50, is transported along an approximately Ω-shaped transporting route due to the rotation operations of these three rollers 51, 55, and 58. Specifically, the sheet-like member 30mf is first transported along an arc-shaped first transporting route R51 for becoming wrapped around the upstream roller 51, then transported along an arc-shaped second transporting route R55 for becoming wrapped around the intermediate roller 55, and finally transported along an arc-shaped third transporting route R58 for becoming wrapped around the downstream roller 58. Thereafter, the sheet-like member 30mf separates from the downstream roller 58 and is sent to the subsequent slitting step S60.

Note that the upstream roller 51 and the intermediate roller 55 are closest to each other at a predetermined position P51 in a rotation direction Dc51 of the upstream roller 51, and this closest position P51 is the position of switching from the first transporting route R51 to the second transporting route R55. Similarly, the intermediate roller 55 and the downstream roller 58 are closest to each other at a predetermined position P55 in a rotation direction Dc55 of the intermediate roller 55, and this closest position P55 is the position of switching from the second transporting route R55 to the third transporting route R58.

The intermediate roller 55 has multiple pin members 55p, 55p... (press-in members) that project from the outer circumferential surface. The pin members 55p, 55p... are members that have a tapered shape on the tip side. Specifically, as shown in FIG. 12, in this example, the pin members 55p each include a conical portion 55pa on the tip side, and a circular column portion 55pb, which has the same diameter as the bottom face of the conical portion 55pa, on the base side, and these portions are integrated with each other. Also, hole portions 51h, 51h... capable of receiving insertion of the pin members 55p, 55p... are provided in the outer circumferential surface of the upstream roller 51. Specifically, the hole portions 51h have a larger diameter than the conical portions 55pa of the pin members 55p in the range of insertion of the pin members 55p. Also, the hole portions 51h are formed so as to correspond to the pin members 55p, such that only one pin member 55p is inserted into each hole portion 51h at the above-described closest position P51.

Accordingly, when the formation target portion, where the air holes h are to be formed, of the sheet-like member 30mf located on the upstream roller 51 shown in FIG. 11 passes the closest position P51, corresponding pin members 55p are inserted into the hole portions 51h of the upstream roller 51 as shown in FIG. 12. Therefore, the pin members 55p are smoothly pressed into the formation target portion of the sheet-like member 30mf, thus swiftly forming the air holes h in the formation target portion.

It should be noted that in this example, given that the air holes h are formed at the closest position P51 as described above, the closest position P51 will hereinafter also be called the "air-hole formation position P51 (corresponding to a through-hole formation position)".

Here, as previously described with reference to FIG. 1, at the time immediately before the air holes h are formed, the pin members 55p are located on the first nonwoven fabric sheet 30mf1 side, not on the second nonwoven fabric sheet 30mf2 side, in the thickness direction of the sheet-like member 30mf. For this reason, when the air holes are formed in the sheet-like member 30mf, as shown in FIG. 2A, the pin members 55p are pressed into the sheet-like member 30mf from the non-facing surface 30mflsn side, which is the one of the two surfaces 30mflsn and 30mflsm of the first nonwoven fabric sheet 30mf1 that does not face the second nonwoven fabric sheet 30mf2. After this pressing, as shown in FIG. 2B, the pin members 55p are pulled out toward the non-facing surface 30mflsn, and therefore the through holes h are formed in the sheet-like member 30mf.

Note that at this time, as shown in FIG. 2A, burrs B2, which project outward in the thickness direction, are formed on the non-facing surface 30mf2sn, which is the one of the two surfaces 30mf2sn and 30mf2sm of the second nonwoven fabric sheet 30mf2 that does not face the first nonwoven fabric sheet 30mf1, and in addition, burrs B1, which project outward, are also formed on the non-facing surface 30mflsn of the first nonwoven fabric sheet 30mf1 as shown in FIG. 2B.

Specifically, as shown in FIG. 2A, when the pin members 55p are pressed in, mainly fibers f2 of the second nonwoven fabric sheet 30mf2 cling to the pin members 55p and move toward the press-in side in the thickness direction, and these fibers project out from the non-facing surface 30mf2sn of the second nonwoven fabric sheet 30mf2 and form the burrs B2. And as shown in FIG. 2B, when the pin members 55p are pulled out as well, mainly fibers f1 of the first nonwoven fabric sheet 30mf1 clinging to the pin members 55p move together therewith toward the non-facing surface 30mflsn, which is the pull-out direction. For this reason, when the pin members 55p are pulled out, the fibers f1 clinging thereto project outward in the thickness direction from the non-facing surface 30mflsn of the first nonwoven fabric sheet 30mf1, and as a result, the outward-projecting burrs B1 are formed on the non-facing surface 30mf1sn of the first nonwoven fabric sheet 30mf1 as well. It should be noted that there is a risk of these burrs B2 and B1 impairing the texture and appearance of the diaper 1.

In view of this, in the present embodiment, an innovation has been made to reduce the size of the burrs B1 that project from the non-facing surface 30mflsn of the first nonwoven fabric sheet 30mf1 and the burrs B2 that project from the non-facing surface 30mf2sn of the second nonwoven fabric sheet 30mf2.

This innovation will be described below. Here, it takes advantage of the fact that the so-called nonwoven fabric sheets constituting the first and second nonwoven fabric sheets 30mf1 and 30mf2 have anisotropy in the thickness direction. Specifically, the innovation takes advantage of the fact that the distribution density (g/cm³) of the fibers of the nonwoven fabric sheets is different on one side of the nonwoven fabric sheet from the other side, and this anisotropy in the thickness direction is formed in the nonwoven-fabric-sheet producing step. For this reason, the nonwoven-fabric-sheet producing step will be described before a description of the innovation is given. Note that this nonwoven-fabric-sheet producing step is substantially the same for both the first nonwoven fabric sheet 30mf1 and the second nonwoven fabric sheet 30mf2. For this reason, the following describes a producing step S200 for producing the first nonwoven fabric sheet 30mf1 as a representative of the two nonwoven fabric sheets 30mf1 and 30mf2.

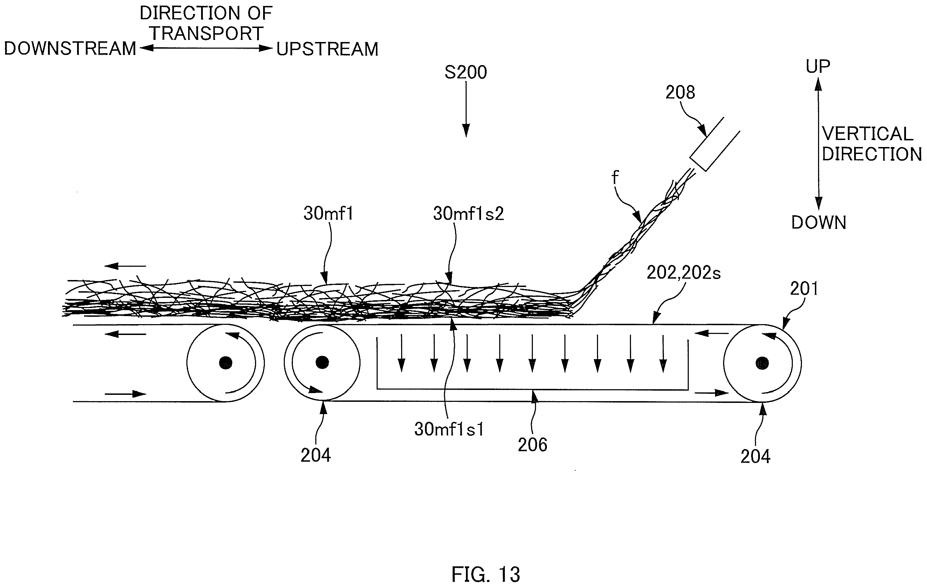

FIG. 13 is a schematic side view of the producing step S200 for producing the first nonwoven fabric sheet 30mf1. As shown in FIG. 13, a suction-belt conveyer 201 is used in the nonwoven-fabric-sheet producing step S200. The suction-belt conveyer 201 has an endless net 202 (corresponding to a support member) that is driven to circulate along a circulation path having a flat route that extends in the direction of transport, and also has a pair of rollers 204 that are arranged on two sides in the direction of transport with the net 202 wrapped thereon. When at least one roller 204 is driven to rotate, an upper surface 202s (corresponding to a support surface) of the net 202 moves in the direction of transport as a flat transporting route.

Also, a suction box 206, which is connected to an appropriate negative pressure source (not shown) such as a blower, is arranged inward of the circulation path of the net 202, and this box 206 can suction air through holes in the upper portion. Due to this suctioning, suction force is generated at the upper surface 202s of the net 202. Accordingly, when melt-spun fibers f are discharged from a nozzle 208 that is arranged above the upper surface 202s of the net 202, the fibers f are suctioned toward and deposited on the upper surface 202s of the net 202, and as a result, the first nonwoven fabric sheet 30mf1 is roughly formed.

It should be noted that when the fibers are deposited on the upper surface 202s of the net 202, more suction force is exerted closer to the upper surface 202s, and the amount of suction force decreases when moving upward away from the upper surface 202s. For this reason, the extent of close-packing of fibers is high at the upper surface 202s of the net 202, and this extent of close-packing of fibers decreases when moving upward away from the upper surface 202s. Also, the deposited direction of the fibers, which is the up-down direction, is also the thickness direction of the first nonwoven fabric sheet 30mf1. For this reason, the first nonwoven fabric sheet 30mf1 has the following anisotropy in the thickness direction: the distribution density (g/cm³) of fibers is high at the surface 30mf1s1 (hereinafter, also called a net-contacting surface 30mf1s1) that is the one of the two surfaces 30mf1s1 and 30mfls2 of the first nonwoven fabric sheet 30mf1 and that comes into contact with the upper surface 202s of the net 202; and the distribution density of fibers is low at the surface 30mfls2 on the other side (corresponding to the non-contact surface, and hereinafter also called a non-net-contacting surface 30mfls2).

Meanwhile, when the fibers of the first nonwoven fabric sheet 30mf1 having such anisotropy are subjected to pressing force in the thickness direction from the pin members 55p, the fibers at positions with a lower distribution density tend to move more easily in the thickness direction than fibers at positions with a higher distribution density.

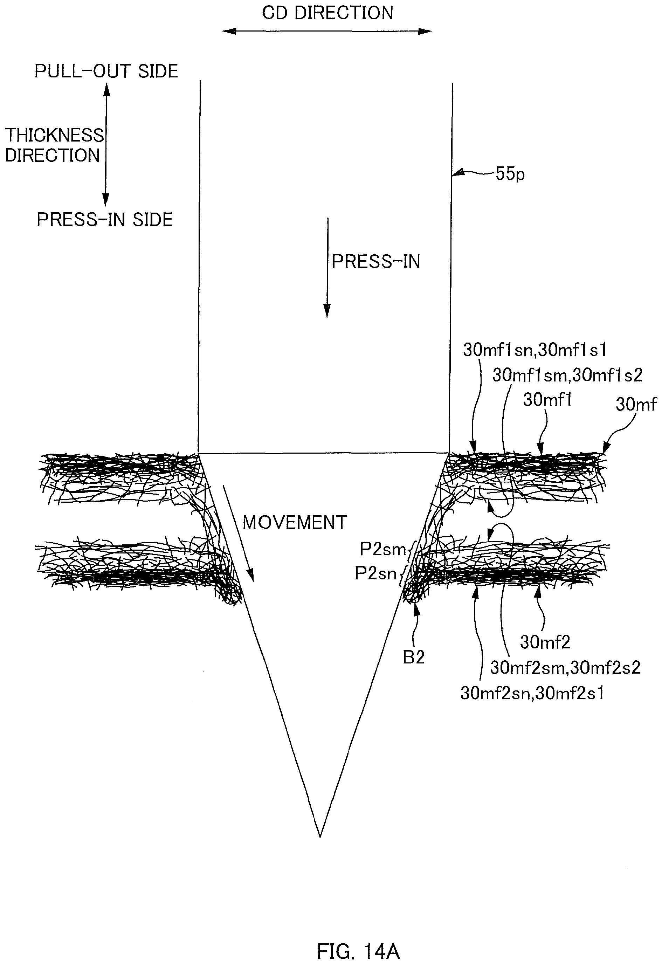

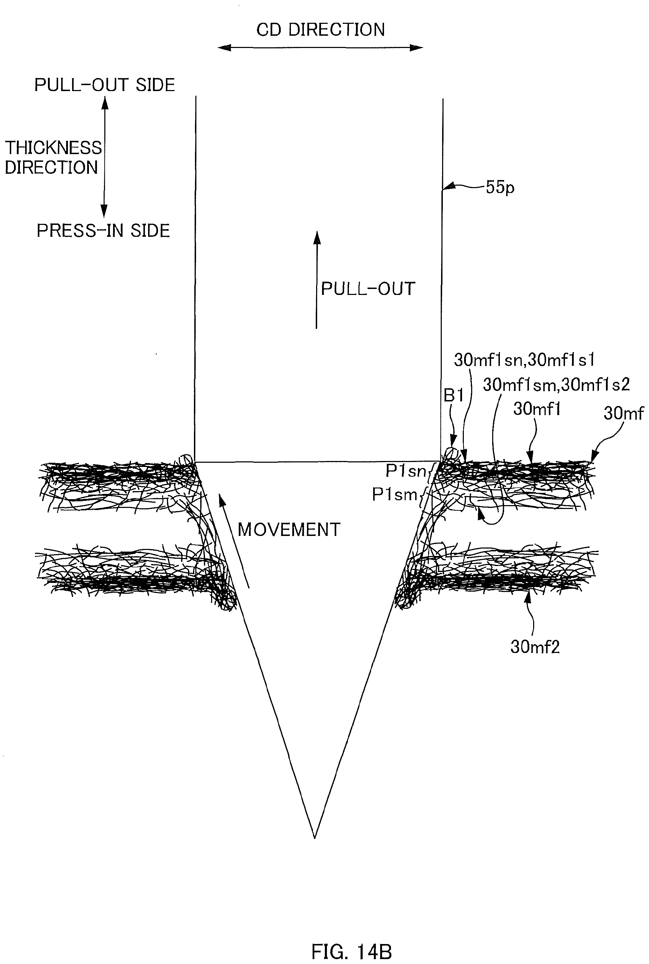

In view of this, in the present embodiment, as shown in FIGS. 14A and 14B, there is an innovation for reducing the size of the burrs B1 that project from the non-facing surface 30mflsn, which is the one of the two surfaces 30mflsn and 30mflsm of the first nonwoven fabric sheet 30mf1 that does not face the second nonwoven fabric sheet 30mf2. The innovation is that the net-contacting surface 30mf1s1, which is one of the two surfaces 30mf1s1 and 30mfls2 of the first nonwoven fabric sheet 30mf1, serves as the non-facing surface 30mf1sn. This will be described in detail below.

First, as shown in FIG. 14A, when the pin member 55p is pressed into the first nonwoven fabric sheet 30mf1 from the non-facing surface 30mflsn thereof, the fibers of the first nonwoven fabric sheet 30mf1 cling to the pin member 55p and move to the press-in side in the thickness direction. Also, as shown in FIG. 14B, when the pin member 55p is pulled out after being pressed in, the fibers clinging to the pin member 55p move together therewith toward the non-facing surface 30mflsn, that is to say in the pull-out direction. Here, in the example shown in FIG. 14B, the non-facing surface 30mflsn forms the net-contacting surface 30mflsl, which is one of the two surfaces 30mf1s1 and 30mfls2 of the first nonwoven fabric sheet 30mf1, as described above.

For this reason, the fiber distribution density (g/cm³) is higher in a portion P1sn of the first nonwoven fabric sheet 30mf1 on the non-facing surface 30mflsn side thereof in the thickness direction, and the fiber distribution density is lower in a portion Plsm that is on the facing surface 30mflsm side, which is the opposite side. More accurately, the portion P1sn on the non-facing surface 30mflsn side has a higher fiber distribution density than the portion Plsm on the facing surface 30mflsm side, which is the opposite side. Accordingly, as shown in FIG. 14B, when the pin member 55p that was pressed into the first nonwoven fabric sheet 30mf1 is pulled out, the fibers in the portion Plsm on the facing surface 30mflsm side easily move in the pull-out direction together with the pin member 55p. But, the fibers in the portion P1sn on the non-facing surface 30mflsn side, which has a higher distribution density, are located on the pull-out side (upper side in FIG. 14B) of the portion P1sm. For this reason, the fibers in the portion P1sn on the non-facing surface 30mflsn side, which are less likely to move in the thickness direction, effectively prevent the fibers in the portion Plsm on the facing surface 30mflsm side from moving in the pull-out direction when they attempt to move in the thickness direction together with the pin member 55p, thus suppressing the case where the burr B1 projects from the non-facing surface 30mflsn.

Also, in this example, the following configuration is employed as an innovation for reducing the size of the second burr B2 shown in FIG. 14A, that is to say the burr B2 that can project from the non-facing surface 30mf2sn, which is the one of the two surfaces 30mf2sn and 30mf2sm of the second nonwoven fabric sheet 30mf2 that does not face the first nonwoven fabric sheet 30mf1.

Specifically, the surface 30mf2s1, which is the one of the two surfaces 30mf2s1 and 30mf2s2 constituting the second nonwoven fabric sheet 30mf2 that comes into contact with the upper surface 202s of the net 202 (hereinafter, also called the net-contacting surface 30mf2s1), also serves as the non-facing surface 30mf2sn of the second nonwoven fabric sheet 30mf2. According to this configuration, projection of the burr B2 from the non-facing surface 30mf2sn is suppressed as described below.

First, as shown in FIG. 14A, the pin member 55p is pressed into the first nonwoven fabric sheet 30mf1, and is then pressed into the second nonwoven fabric sheet 30mf2. When being pressed into the second nonwoven fabric sheet 30mf2, the pin member 55p is pressed into, first, the portion P2sm on the facing surface 30mf2sm side, and then pressed into the portion P2sn on the non-facing surface 30mf2sn side. In other words, the pin member 55p is pressed into the portion P2sn on the non-facing surface 30mf2sn side of the second nonwoven fabric sheet 30mf2 last. At this time, when the fibers clinging to the pin member 55p move together therewith in the direction of press-in, the burr B2 is formed as a portion that projects outward from the non-facing surface 30mf2sn.

However, the non-facing surface 30mf2sn of the second nonwoven fabric sheet 30mf2 is the above-described net-contacting surface 30mf2s1, and due to the configuration described above, the distribution density of fibers in the net-contacting surface 30mf2s1 is high. In other words, the fiber distribution density is high in the portion P2sn on the non-facing surface 30mf2sn side. Accordingly, the fibers in this portion P2sn on the non-facing surface 30mf2sn side are not likely to move in the thickness direction, which is the direction of press-in, and this consequently suppresses the projection of the burr B2 from the non-facing surface 30mf2sn of the second nonwoven fabric sheet 30mf2.

Note that in the case where the burr B2 at the non-facing surface 30mf2sn of the second nonwoven fabric sheet 30mf2 does not cause a big problem, the positional relationship in the thickness direction in the second nonwoven fabric sheet 30mf2 may be set to the opposite of the above-described relationship. Specifically, the net-contacting surface 30mf2s1 of the second nonwoven fabric sheet 30mf2 may be formed so as to correspond to the facing surface 30mf2sn of the second nonwoven fabric sheet 30mf2.

Also, as can be understood from a comparison of FIGS. 4, 9B, and the enlarged view of relevant portions in FIG. 12, in this example, the non-facing surface 30mflsn of the first nonwoven fabric sheet 30mf1 ultimately forms the non-skin-side surfaces of the front band member 31 and the back band member 41 in the diaper 1. For this reason, reducing the size of the burrs B1 on the non-facing surface 30mflsn as described above effectively contributes to giving the air holes h a nice appearance when the front band member 31 and the back band member 41 are viewed from the non-skin side. Accordingly, a diaper 1 that has a favorable appearance can be manufactured.

Note that there is no limitation whatsoever to this. Specifically, the non-facing surface 30mflsn of the first nonwoven fabric sheet 30mf1 may ultimately form the skin-side surfaces of the front band member 31 and the back band member 41. With this configuration, reducing the size of the burrs B1 on the non-facing surface 30mflsn as described above effectively contributes to providing a favorable texture when the front band member 31 and the back band member 41 are touched on the skin side. Accordingly, a diaper 1 that has a favorable texture can be manufactured.

Furthermore, in this example, as previously described, a hot-melt adhesive is used for fixing the elastic-string continuous bodies 35a and 45a to the first nonwoven fabric sheet 30mf1 and the second nonwoven fabric sheet 30mf2, and the hot-melt adhesive is applied to the elastic-string continuous bodies 35a and 45a. Accordingly, the first nonwoven fabric sheet 30mf1 and the second nonwoven fabric sheet 30mf2 are fixed to each other with the elastic-string continuous bodies 35a and 45a, or in other words, the hot-melt adhesive applied to the elastic-string continuous bodies 35a and 45a functions as fixing portions for fixing the first nonwoven fabric sheet 30mf1 and the second nonwoven fabric sheet 30mf2 to each other. For this reason, in this example, not provided are direct fixing portions which are for directly fixing the first nonwoven fabric sheet 30mf1 and the second nonwoven fabric sheet 30mf2 to each other. In other words, a hot-melt adhesive is not applied to the first nonwoven fabric sheet 30mf1 and the second nonwoven fabric sheet 30mf2 as direct fixing portions. And, not provided are welding portions which are for directly fixing the first nonwoven fabric sheet 30mf1 and the second nonwoven fabric sheet 30mf2 by welding. Accordingly, the softness of the sheet-like member 30mf is improved.

Also, as shown in FIG. 9B, in this example, the air holes h are formed at a position between pairs of the elastic-string continuous bodies 35a, 35a (45a, 45a) in the sheet-like member 30mf. For this reason, the air holes h are formed at positions where the hot-melt adhesive, which serves as fixing portions, is not provided, that is to say the air holes h are formed so as to not be overlapped with the fixing portions. Accordingly, it is possible to effectively prevent a problem that can occur if the air holes h are formed so as to be overlapped with the fixing portions, such as the problem of difficulty in punching the air holes h due to an increase in punching resistance caused by the fixing portions when punching the air holes h, and the problem where the softness of the sheet-like member 30mf is impaired by the hardening of the burrs B1 and B2 caused by the fixing portions.

Note that there is no limitation whatsoever to this. For example, if a certain extent of hardening of the sheet-like member 30mf is not a problem, a configuration is possible in which the first nonwoven fabric sheet 30mf1 and the second nonwoven fabric sheet 30mf2 are directly fixed to each other by applying a hot-melt adhesive to at least either the first nonwoven fabric sheet 30mf1 or the second nonwoven fabric sheet 30mf2 instead of or in addition to the hot-melt adhesive applied to the elastic-string continuous bodies 35a and 45a. In other words, the previously described direct fixing portions may be provided. However, in this case as well, it is desirable that the air holes h are formed at positions where the hot-melt adhesive, which serves as the direct fixing portions, is not provided. In other words, it is desirable that the air holes h are formed so as to not be overlapped with positions where the hot-melt adhesive is applied in the first nonwoven fabric sheet 30mf1 and the second nonwoven fabric sheet 30mf2. According to this configuration, it is possible to achieve actions and effects similar to those described above, that is to say being able to prevent an increase in punching resistance of the air holes h and hardening of the burrs B1 and B2. Note that the direct fixing portions for directly fixing the first nonwoven fabric sheet 30mf1 and the second nonwoven fabric sheet 30mf2 to each other without using the elastic-string continuous bodies 35a and 45a is not limited to the above-described example of a hot-melt adhesive. For example, the concept of the direct fixing portions also includes the previously described welding portions for welding the first nonwoven fabric sheet 30mf1 and the second nonwoven fabric sheet 30mf2 to each other.

Also, the apex angle of the conical portion 55pa in FIG. 12 is selected from the range of 20° to 45° for example, and is 36° in this example. Also, the height of the conical portion 55pa is selected from the range of 3 mm to 8 mm for example, and is 4.6 mm in this example. Note that there is no limitation whatsoever to this. Furthermore, although the edge portions of the hole portions 51h are chamfered in this example, there is no limitation whatsoever to this, or in other words, the edge portions are not required to be chamfered.

Also, in this example, the intermediate roller 55 in FIG. 11 is a large-diameter roller having a circumferential length that corresponds to approximately two times the above-described product pitch P1, such that two pairs of air hole groups Gh31 and two pairs of air hole groups Gh41 corresponding to two diapers shown in FIG. 9B can be formed over one full rotation. Also, the upstream roller 51 in FIG. 11 is a small-diameter roller having a circumferential length that substantially corresponds to the product pitch such that one pair of air hole groups Gh31 and one pair of air hole groups Gh41 corresponding to one diaper shown in FIG. 9B can be formed over one full rotation. Note that there is no limitation whatsoever to this.

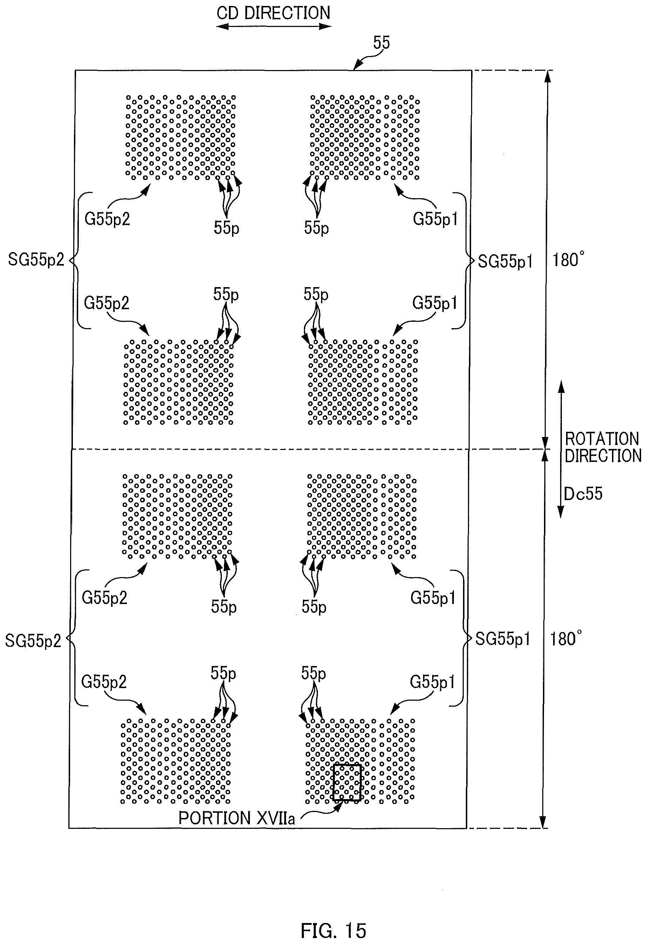

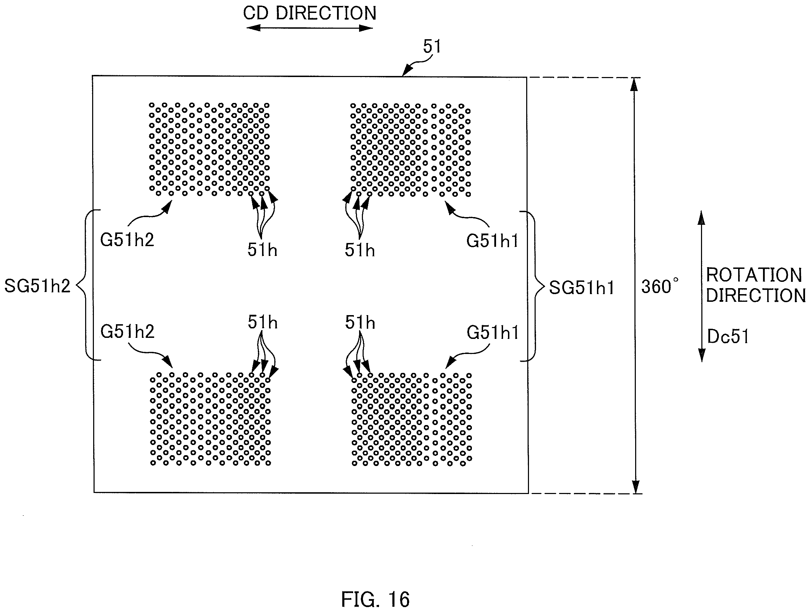

FIG. 15 is a diagram illustrating an arrangement pattern of the pin members 55p on the outer circumferential surface of the intermediate roller 55, and FIG. 16 is a diagram illustrating an arrangement pattern of the hole portions 51h on the outer circumferential surface of the upstream roller 51. Note that these diagrams both show the outer circumferential surfaces of the rollers 55 and 51 in a state of being spread flat. Also, later-described receiving portions 55r are not shown in FIG. 15 in order to prevent complexity in the drawing.

As shown in FIG. 15, the pin members 55p are provided in correspondence with the air hole groups Gh31 and Gh41 that are formed in the previously described sheet-like member 30mf. Specifically, as shown in FIG. 9B, in the region of the sheet-like member 30mf that is on the one side in the CD direction, one pair of air hole groups Gh31 for the front band member 31 is to be formed in each diaper, and therefore, as shown in FIG. 15, in the region of the outer circumferential surface of the intermediate roller 55 that is on the one side in the CD direction, a pair of pin member groups G55p1 are provided side-by-side in the rotation direction Dc55, each having pin members 55p in the same staggered arrangement as the air hole groups Gh31. Similarly, in the region of the sheet-like member 30mf that is on the other side in the CD direction, one pair of air hole groups Gh41 for the back band member 41 is to be formed in each diaper, and therefore, in the region of the outer circumferential surface of the intermediate roller 55 that is on the other side in the CD direction, a pair of pin member groups G55p2 are provided side-by-side in the rotation direction Dc55, each having pin members 55p in the same staggered arrangement as the air hole groups Gh41.

Also, in this example, as previously described, the intermediate roller 55 is provided with air holes h corresponding to two diapers for each full rotation. Accordingly, letting one pin member group set SG55p1 (SG55p2) be one set of two pin member groups G55p1, G55p1 (G55p2, G55p2) that are side-by-side in the rotation direction Dc55, the outer circumferential surface of the intermediate roller 55 is provided with two pin member group sets SG55p1, SG55p1 (SG55p2, SG55p2) that are side-by-side at an even pitch of 180° in the direction of rotation Dc55.

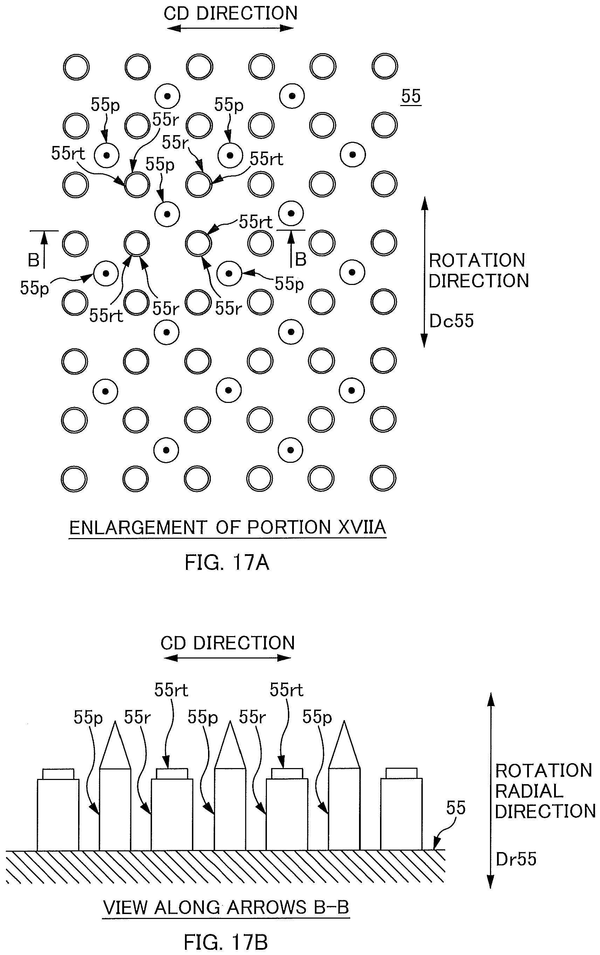

Furthermore, FIG. 17A shows an enlarged view of a portion XVIIa in FIG. 15, and FIG. 17B shows a view taken along arrows B-B in FIG. 17A. As shown in FIG. 17A, receiving portions 55r that are separate from the pin members 55p are formed in the outer circumferential surface of the intermediate roller 55 as projections at positions between adjacent pin members 55p. Also, as shown in FIG. 17B, the receiving portions 55r are each an approximately circular columnar body that has, at the leading end, a top surface 55rt that faces outward in a rotation radial direction Dr55 of the intermediate roller 55. Accordingly, the sheet-like member 30mf is received and supported by not only the pin members 55p, but also the receiving portions 55r. For this reason, the sheet-like member 30mf can overall be stably supported at an appropriate position in the rotation radial direction Dr55 of the intermediate roller 55. Also, at this time, the top surfaces 55rt of the receiving portions 55r receive the one surface of the sheet-like member 30mf without penetrating it, and thus also effectively contributes to stably supporting the sheet-like member 30mf at the appropriate position.

Also, in this example, as shown in FIG. 17A, the receiving portions 55r are provided at four locations around each pin member 55p, thus making it possible to reliably receive the sheet-like member 30mf, but there is no limitation whatsoever to the aforementioned four locations. For example, if arrangement space cannot be ensured, the number of locations may be smaller, or on the other hand, if arrangement space can be ensured, the number of locations may be larger.

Furthermore, in the example shown in FIG. 17B, the positions of the top surfaces 55rt of the receiving portions 55r are inward in the rotation radial direction Dr55 of the intermediate roller 55 relative to the positions of the apexes of the pin members 55p. This therefore prevents the receiving portions 55r from hindering the processing for forming the air holes h with use of the pin members 55p. Note that here, it is desirable that the top surface 55rt of the receiving portion 55r is located in the vicinity of the bottom face of the conical portion 55pa of the pin member 55p, and for example, it is desirable that the position of the top surface 55rt in the rotation radial direction Dr55 is in a range of ±2 mm centered about the position of the bottom face. In this example, the top surface 55rt is matched with the bottom face of the conical portion 55pa. Accordingly, the receiving portions 55r can reliably receive the sheet-like member 30mf without hindering the processing for forming the air holes h with use of the pin member 55p. Accordingly, the pin members 55p can swiftly form the air holes h in the sheet-like member 30mf.

Also, in this example, the top surface 55rt of the receiving portion 55r is shaped as a perfect circle having a diameter selected from the range of 2 mm to 5 mm for example, but there is no limitation whatsoever to this. For example, it may be shaped as a polygon such as an equilateral triangle or a square, or may have another shape.

Also, as shown in FIG. 16, the hole portions 51h in the outer circumferential surface of the upstream roller 51 are provided in correspondence with the pin members 55p. Specifically, as shown in FIG. 15, the two pin member groups G55p1 are provided side-by-side in the rotation direction Dc55 in the region of the outer circumferential surface of the intermediate roller 55 that is on the one side in the CD direction, and therefore in correspondence with this, two hole portion groups G51h1, each of which has multiple hole portions 51h in a staggered arrangement, are provided side-by-side in the rotation direction Dc51 in the region of the outer circumferential surface of the upstream roller 51 that is on the one side in the CD direction. Similarly, as shown in FIG. 15, the two pin member groups G55p2 are provided side-by-side in the rotation direction Dc55 in the region of the outer circumferential surface of the intermediate roller 55 that is on the other side in the CD direction, and therefore in correspondence with this, two hole portion groups G51h2, each of which has multiple hole portions 51h in a staggered arrangement, are provided side-by-side in the rotation direction Dc51 in the region of the outer circumferential surface of the upstream roller 51 that is on the other side in the CD direction.

It should be noted that as previously described, the upstream roller 51 contributes to forming air holes h corresponding to one diaper for each full rotation, and therefore letting one hole portion group set SG51h1 (SG51h2) be one set of two hole portion groups G51h1, G51h1 (G51h2, G51h2) that are side-by-side in the rotation direction Dc51, the outer circumferential surface of the upstream roller 51 is provided with only one hole portion group set SG51h1 (SG51h2).

Note that in some cases, a heating device (not shown) such as an electrothermal heater is provided in the intermediate roller 55 in FIG. 11, and therefore the pin members 55p of the intermediate roller 55 may be heated. According to this configuration, the thermoplastic resin fibers that constitute the nonwoven fabric sheets 30mf1 and 30mf2 of the sheet-like member 30mf can be made softer with use of the heated pin members 55p. Accordingly, the pin members 55p can easily push the fibers to the side when being pressed into the sheet-like member 30mf, thus more easily forming the air holes h.

It should be noted that one example of standard for heating is that the temperature of the pin members 55p is greater than or equal to the softening point of the thermoplastic resin and less than the melting point thereof. The softening point can be obtained by TMA (thermomechanical analysis) in accordance with JIS K 7196 (Testing Method for Softening Temperature of Thermoplastic Film and Sheeting by Thermomechanical Analysis) . Also, the melting point can be obtained as the melting peak temperature in DSC (differential scanning calorimetry) in accordance with JIS K 7121 (Testing Methods for Transition Temperatures of Plastics).

Also, the configuration of the downstream roller 58 in FIG. 11 is substantially the same as that of the upstream roller 51. For example, the outer circumferential surface of the downstream roller 58 is provided with hole portion groups G58h1 and G58h2 that have the same specifications as the hole portion groups G51h1 and G51h2 in the outer circumferential surface of the upstream roller 51. Accordingly, when the sheet-like member 30mf is received from the intermediate roller 55, the pin members 55p of the intermediate roller 55 are smoothly inserted into hole portions 58h in the hole portion groups G58h1 and G58h2, thus making it possible to receive the sheet-like member 30mf without largely deforming the shape of the air holes h formed in the sheet-like member 30mf.

### Slitting step S60

As shown in FIG. 8, in the slitting step S60, the sheet-like member 30mf, which is sent from the air-hole forming step S50, is divided into two portions at a boundary position BL (FIG. 9B) between the regions on the one side and the other side in the CD direction. Accordingly, the front-band-member continuous body 31a, in which the elastic-string continuous bodies 35a are uncut, and the back-band-member continuous body 41a, in which the elastic-string continuous bodies 45a are uncut, are produced side-by-side in the CD direction (FIG. 8).

This dividing processing can be carried out using a known slitter device 60 shown in FIG. 8. Specifically, for example, this device 60 has a pair of upper and lower disc-shaped rotary blades, and divides the sheet-like member 30mf into two portions in the CD direction using the blade edges at the peripheral edges of the rotating blades. This type of device 60 is well-known. Accordingly, this will not be described further.

### Gap forming step S70

As shown in FIG. 8, in the gap forming step S70, the front-band-member continuous body 31a and the back-band-member continuous body 41a, which are sent from the slitting step S60, are each moved outward in the CD direction, thus forming a gap between the continuous bodies 31a and 41a in the CD direction, and this gap has a size corresponding to the longitudinal gap Ld between the front band member 31 and the back band member 41 in the diaper 1 in the unfolded state in FIG. 4.

Note that the movement of the front-band-member continuous body 31a outward in the CD direction and the movement of the back-band-member continuous body 41a outward in the CD direction can be realized by arranging multiple transporting rollers (not shown) along the transporting route in consideration of such movement, and the detail of such a configuration can be arrived at based on the above description. Accordingly, this will not be described further.

### Front elastic string cutting step S80

In the front-elastic-string cutting step S80, the front-band-member continuous body 31a is sent from the gap forming step S70 and is shown in FIG. 10A. As shown in FIG. 10B, in the front-band-member continuous body 31a, the elastic-string continuous bodies 35a are cut in the non-fixed region AL1 that corresponds to the non-stretchy region AL, and the elastic-string continuous bodies 35a are not cut in the fixed regions AH1 that correspond to the stretchy regions AH. Accordingly, the non-stretchy region AL and the stretchy regions AH are formed in the front-band-member continuous body 31a. Note that the region AL1 corresponding to the non-stretchy region AL appears at the product pitch P1 of the diaper 1 in the direction of transport. Accordingly, this cutting is performed on the front-band-member continuous body 31a at the product pitch P1 of the diaper 1.

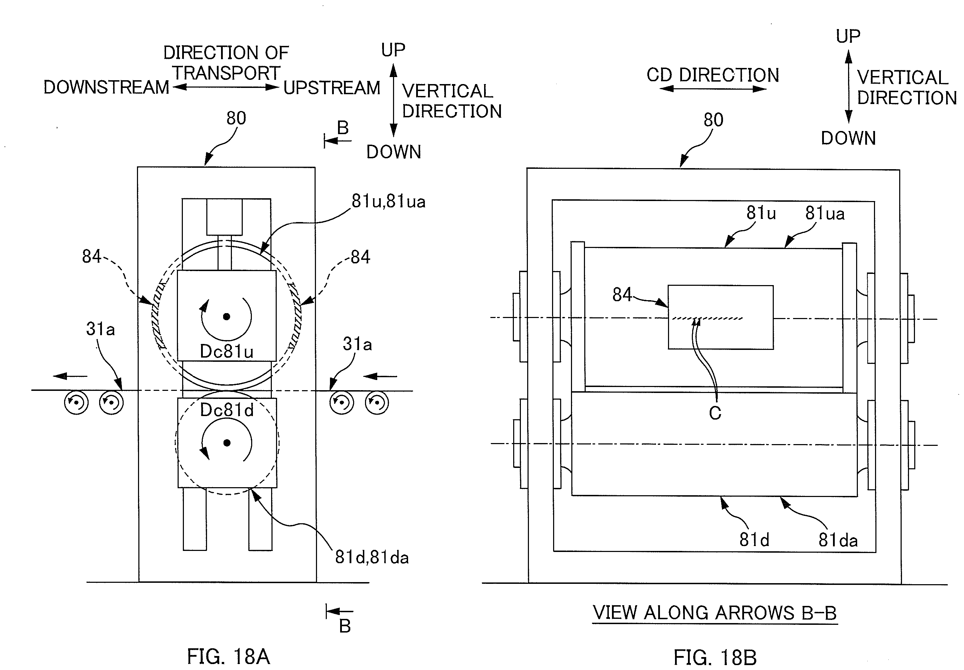

This cutting is performed by a front-side cutter device 80. FIG. 18A is a schematic side view of this cutter device 80, and FIG. 18B is a view taken along arrows B-B in FIG. 18A.

The front-side cutter device 80 has a pair of upper and lower rollers 81u and 81d that are driven to rotate about rotation axes that extend in the CD direction, and have outer circumferential surfaces 81ua and 81da that face each other. Also, projecting cutter blades C, C... are formed on a cutter block 84 on the outer circumferential surface 81ua of the upper roller 81u at positions corresponding to predetermined positions PC shown in FIG. 10B. The outer circumferential surface 81da of the lower roller 81d is formed as a smooth surface in order to receive the cutter blades C, C.... Also, these rollers 81u and 81d are controlled to rotate in coordination with the operation for transporting the front-band-member continuous body 31a, and therefore the cutter roll 81u rotates such that each time the previously described non-fixed region AL1 corresponding to the non-stretchy region AL passes, the cutter block 84 faces the non-fixed region AL1. Accordingly, each time the non-fixed region AL1 in FIG. 10A passes between the outer circumferential surfaces 81ua and 81da of the pair of rollers 81u and 81d, the non-fixed region AL1 is pressed by the cutter blades C, C... on the cutter block 84 of the cutter roll 81u and the outer circumferential surface 81da of the anvil roll 81d. The elastic-string continuous bodies 35a, 35a... are thus cut at the pressed positions PC.

### Back elastic string cutting step S90

As shown in FIG. 8, in the back-elastic-string cutting step S90, cutting that is similar to the processing described with reference to FIGS. 10A and 10B is likewise performed on the back-band-member continuous body 41a that is sent from the gap forming step S70. Specifically, the elastic-string continuous bodies 45a are cut in the region AL1 corresponding to the non-stretchy region AL, and the elastic-string continuous bodies 45a are not cut in the regions AH1 corresponding to the stretchy regions AH. Accordingly, the non-stretchy region AL and the stretchy regions AH are formed in the back-band-member continuous body 41a. Note that as can be understood from the above description, this cutting is substantially the same as the previously described front-elastic-string cutting step S80, and the configuration of a back-side cutter device 90, which is shown in FIG. 8 and is for performing this cutting processing, is also the same as the front-side cutter device 80. Accordingly, a detailed description will not be given for it here.

### Absorbent-main-body attaching step S200

As shown in FIG. 8, in the absorbent-main-body attaching step S200, the cutform-shaped absorbent main body 10, which is produced in other steps that are not shown, is arranged spanning between and fixed to the band member continuous bodies 31a and 41a that are received as the intermediate product 1m from the elastic string cutting steps S80 and S90, and therefore the intermediate product 1m becomes the approximately ladder-shaped diaper continuous body 1Ha that is formed by a series of diapers 1H that are unfolded and approximately H-shaped as shown in FIG. 4.

Note that this processing for attaching the absorbent main body 10 can be realized using, for example, a rotating drum (not shown) in which holding portions for suctioning and holding the absorbent main body 10 to the outer circumferential surface are provided side-by-side at the product pitch P1 in the rotation direction, and this rotating drum is well-known. Accordingly, a detailed description will not be given for it.

### Step S210 located upstream from air-hole forming step S50

In the embodiment described above, only the air-hole forming step S50 and the steps S60 to S130 at positions downstream thereof are described, and a step S210 located upstream therefrom is not described. Accordingly, the following describes this upstream step S210.

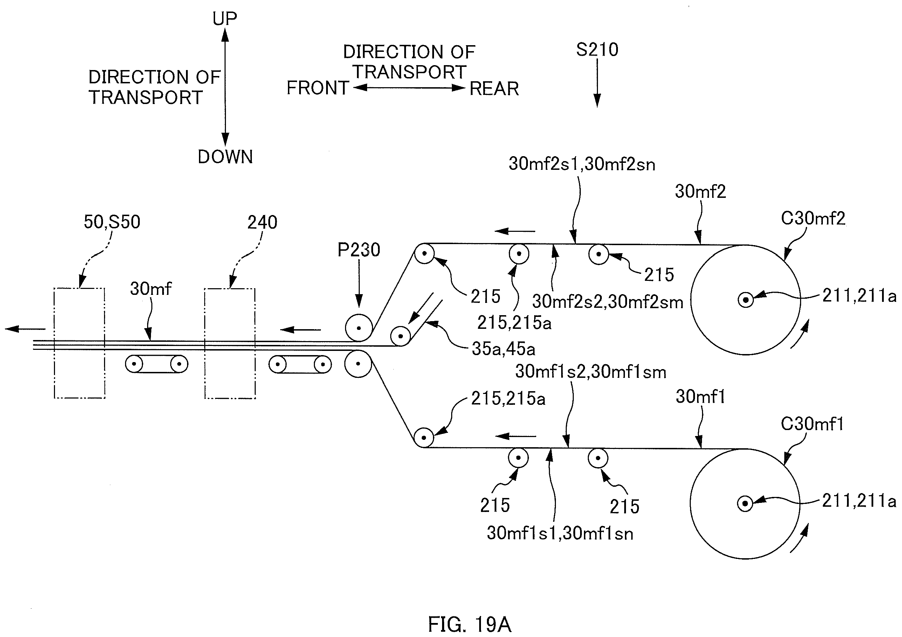

FIG. 19A is a schematic side view of the step S210 performed at a location that is upstream, with respect to the direction of transport, of the position corresponding to the air-hole forming step S50 in the manufacturing line.

As shown in FIG. 19A, first, the first nonwoven fabric sheets 30mf1 and the second nonwoven fabric sheets 30mf2 are carried in the form of material coils C30mf1 and C30mf2, to this manufacturing line from the nonwoven-fabric-sheet producing step S200, which has already been described with reference to FIGS. 13 and the like. Here, as shown in FIG. 19A, the material coils C30mf1 and C30mf2 are obtained by the first nonwoven fabric sheets 30mf1 and the second nonwoven fabric sheets 30mf2 being wound into a coil in the direction in which the nonwoven fabric sheets 30mf1 and 30mf2 are continuous. The material coils C30mf1 and C30mf2 are respectively attached to rotation shafts 211a of two corresponding feeding devices 211. Accordingly, when the two rotation shafts 211a are driven to rotate, the first nonwoven fabric sheets 30mf1 and the second nonwoven fabric sheets 30mf2 are fed from the material coils 30mf1 and 30mf2 and transported along the direction of transport.

The first nonwoven fabric sheets 30mf1 and the second nonwoven fabric sheets 30mf2, which are fed from the material coils C30mf1 and C30mf2, then merge at a predetermined merging position P230 in the direction of transport. Also, at the merging position P230, the elastic-string continuous bodies 35a and 45a, which are stretched in the direction of transport, are transported along to the direction of transport toward a position between the first nonwoven fabric sheet 30mf1 and the second nonwoven fabric sheet 30mf2, and a hot-melt adhesive has been applied to the elastic-string continuous bodies 35a and 45a in advance. Accordingly, the continuous bodies 35a and 45a are fixed to the first nonwoven fabric sheet 30mf1 and the second nonwoven fabric sheet 30mf2 using the hot-melt adhesive, and the first nonwoven fabric sheet 30mf1 and the second nonwoven fabric sheet 30mf2 are fixed to each other via the elastic-string continuous bodies 35a and 45a.

Thereafter, using a known folding device 240 that is arranged downstream of the merging position P230 in the direction of transport, the end portions 30mf1p1 and 30mflp2 of the first nonwoven fabric sheet 30mf1 in the CD direction are each folded inward in the CD direction as shown in FIG. 9A, and these folded portions cover the skin side of the end portions 30mf2p1 and 30mf2p2 of the second nonwoven fabric sheet 30mf2 in the CD direction.

The above thus produces the sheet-like member 30mf in the previously described state immediately before being sent to the air-hole forming step S50 as shown in FIG. 9A.

Here, in this example, the first nonwoven fabric sheets 30mf1 is transported from the feeding device 211 to the merging position P230 by multiple transporting rollers 215, and it is desirable that at least one of these transporting rollers 215 is a transporting roller 215a having the specifications shown in FIG. 19A. Specifically, the transporting roller 215a is a driven roller that is driven to rotate, and it is desirable that this roller 215a comes into contact with the non-net-contacting surface 30mfls2 (the surface 30mfls2 that does not come into contact with the upper surface 202s of the net 202 of the suction-belt conveyer 201 in FIG. 13), which is one of the two surfaces 30mf1s1 and 30mfls2 of the first nonwoven fabric sheet 30mf1, and is thus driven to rotate by rotation force received from the first nonwoven fabric sheet 30mf1.

Also, due to providing this transporting roller 215a, the fibers in the portion of the first nonwoven fabric sheet 30mf1 that is on the non-net-contacting surface 30mfls2 side become further relaxed by shearing force that is applied through contact with the transporting roller 215a. Specifically, this portion is a portion where the fiber distribution density is low, that is to say a portion that is relaxed to a certain extent in the first place, and the fibers become further relaxed due to coming into contact with the transporting roller 215a. This therefore makes it possible to effectively lower the punching resistance when forming the air holes h in the first nonwoven fabric sheet 30mf1, thus making it easier to form the air holes h.

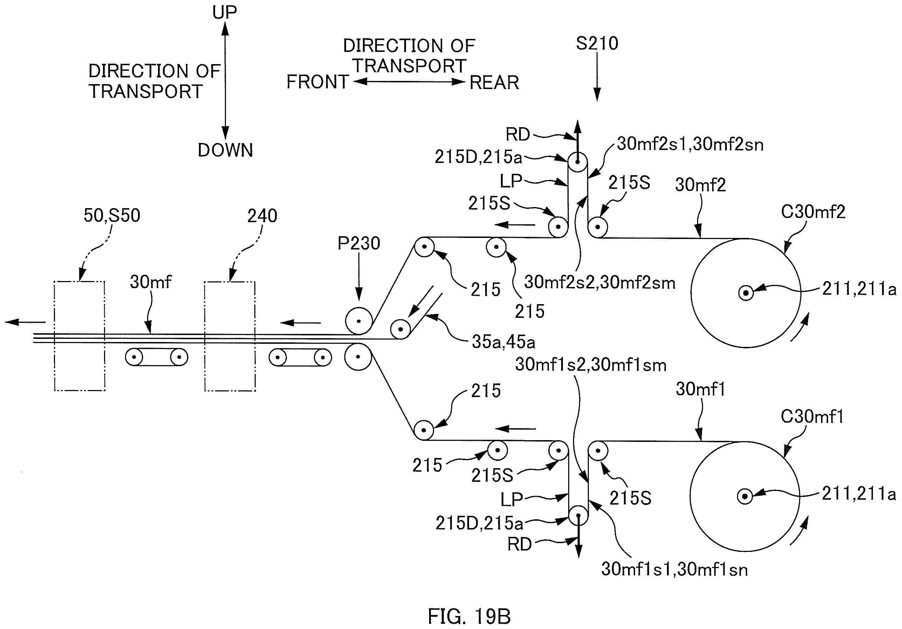

Also, it is more desirable that the transporting roller 215a is not only a driven roller as described above, but also serves as a dancer roller 215D that has a dancer function as shown in FIG. 19B. Note that the dancer roller 215D is used for maintaining a constant tension value (N) for the first nonwoven fabric sheets 30mf1 that are feed from the feeding device 211 for example, and this will be described in detail below.

First, the dancer roller 215D is located at a position between a pair of stationary rollers 215S that rotate at fixed positions, and is guided along and capable of moving back and forth in a predetermined direction (the up-down direction in the example in FIG. 19B) in which the first nonwoven fabric sheets 30mf1 are continuous. Also, the first nonwoven fabric sheets 30mf1 are wound around the pair of stationary rollers 215S and the dancer roller 215D, thus forming a loop LP in the first nonwoven fabric sheets 30mf1, and an appropriate load applying device (not shown) such as an air cylinder or sinker applies a predetermined load RD to the dancer roller 215D in a direction according to which the loop LP increases in size in the predetermined direction. Also, the position of the dancer roller 215D in the predetermined direction is measured by an appropriate sensor (not shown), and an appropriate controller (not shown) such as a sequencer controls the rotation speed value (mpm) of the rotation shaft 211a of the feeding device 211 based on a measurement signal that is output from the sensor. Specifically, if the measurement signal indicates that "the size of the loop LP is smaller than the target value", the controller increases the rotation speed value (mpm) of the rotation shaft 211a, whereas if the measurement signal indicates that "the size of the loop LP is larger than the target value", the controller reduces the rotation speed value (mpm) of the rotation shaft 211a. Accordingly, a constant tension value is maintained for the first nonwoven fabric sheets 30mf1 that are fed from the feeding device 211.

Note that in the case where the transporting roller 215a also serves as the dancer roller 215D in this manner, the predetermined load RD acting in the direction for increasing the size of the loop LP as described above is applied to the dancer roller 215D. For this reason, the previously described shearing force that is applied to the first nonwoven fabric sheets 30mf1 through contact with the dancer roller 215D can be increased by an amount corresponding to the above-described effect of the load RD. Accordingly, it is possible to more effectively relax the fibers in the portion of the first nonwoven fabric sheet 30mf1 that is on the non-net-contacting surface 30mfls2 side with use of this shearing force.

It should be noted that, as shown in FIG. 19A, the transporting roller 215a having the above-described functions may also be provided in the transporting route that is between the feeding device 211 for the second nonwoven fabric sheet 30mf2 and the merging position P230. In other words, at least one of the transporting rollers 215 in this transporting route may be the transporting roller 215a having the above-described functions. In this case, this transporting roller 215a is also a driven roller, and this roller 215a comes into contact with the non-net-contacting surface 30mf2s2 (the surface 30mf2s2 that does not come into contact with the upper surface 202s of the net 202 of the suction-belt conveyer 201 in FIG. 13), which is one of the two surfaces 30mf2s1 and 30mf2s2 of the second nonwoven fabric sheet 30mf2, and is thus driven to rotate by rotation force received from the second nonwoven fabric sheet 30mf2.

Furthermore, a configuration is possible in which the transporting roller 215a for the second nonwoven fabric sheet 30mf2 is not only a driven roller as described above, but also serves as a dancer roller 215D that has a dancer function as shown in FIG. 19B. Note that it is thought that the content of this configuration can be easily arrived at based on the content already described for the first nonwoven fabric sheet 30mf1. For this reason, the same or similar configurations are denoted by the same reference signs, and will not be described further.

### Other embodiments

Although the embodiment of the present disclosure have been described hereinabove, the above embodiment of the present disclosure are simply to facilitate understanding of the present disclosure and are not in any way to be construed as limiting the present disclosure. The present disclosure may variously be changed or altered without departing from its gist and encompass equivalents thereof. For example, deformation which will be described below is possible.

Although the elastic-string continuous bodies 35a and 45a are illustrated as examples of the continuous elastic members in the above embodiment, there is no limitation whatsoever to this. For example, they may be elastic belt continuous bodies.

Each of the pin members 55p of the intermediate roller 55 has the conical portion 55pa and the circular column portion 55pb as shown in the enlarged view of relevant portions in FIG. 12 in the above embodiment, but there is no limitation whatsoever to this. For example, instead of the conical portion 55pa, it may have a pyramid-shaped portion that has a polygonal cross-section, such as a triangular pyramid or a quadrangular pyramid, and instead of the circular column portion 55pb, it may have a columnar portion that has a polygonal cross-section, such as a triangular column or a quadrangular column.

In the above embodiment, the sheet-like member 30mf serving as the intermediate product 1m is wrapped around both the upstream roller 51 and the intermediate roller 55 of the air-hole forming device 50 in the air-hole forming step S50 as shown in FIG. 11, but there is no limitation whatsoever to this. Specifically, the sheet-like member 30mf may pass between the rollers 51 and 55 without being wrapped around the upstream roller 51 and the intermediate roller 55.

Although the three-piece type of disposable diaper 1 is illustrated as an example of the absorbent article in the above embodiment, there is no limitation whatsoever to this. For example, the method and device for manufacturing a sheet-like member of the present invention may be applied when manufacturing a sheet-like member that is a material for constituting a two-piece type of disposable diaper. It should be noted that a two-piece type of disposable diaper is a type of diaper that has an exterior sheet as a first component having a front portion, a crotch portion, and a back portion, and the absorbent main body 10 as second component that is fixed to the skin-side surface of the exterior sheet. Also, in this case, the continuous sheet of exterior sheets is formed by two overlapped nonwoven fabric sheets, and the method and device for manufacturing a sheet-like member of the present invention is used when forming hole portions in the two overlapped nonwoven fabric sheets and cutting elastic members arranged between the two overlapped nonwoven fabric sheets.

Furthermore, this two-piece type of diaper may be a so-called tape-type disposable diaper. Note that a tape-type disposable diaper is a type of diaper that uses fastening tape in order to connect the front portion, which covers the front side of the trunk portion of the wearer, to the back portion, which cover the back side of the trunk portion.

Furthermore, the absorbent article is not limited whatsoever to being the disposable diaper 1. Specifically, the method and device for manufacturing a sheet-like member of the present invention is applied when manufacturing any absorbent article that is manufactured with use of nonwoven fabric sheets. For this reason, the concept of this absorbent article also encompasses a urine absorbing pad, a sanitary napkin, and the like.

### [Reference Signs List]

1 disposable diaper (absorbent article),
1a diaper continuous body, 1Ha approximately ladder-shaped diaper continuous body,
1m intermediate product,
10 absorbent main body,
10LG portion, 10ea end portion, 10eb end portion,
11 absorbent body, 11c absorbent core,
13 top sheet, 15 back sheet,
15a leak-proof sheet, 15b exterior sheet,
16 elastic string,
30mf sheet-like member (composite sheet), 30mfe end portion,
30mf1 first nonwoven fabric sheet,
30mf1p1 portion, 30mflp2 portion,
30mflsn non-facing surface, 30mflsm facing surface,
30mf1s1 net-contacting surface (in-contact surface),
30mfls2 non-net-contacting surface (non-contacting surface, surface on opposite side, not-in-contact surface),
30mf2 second nonwoven fabric sheet,
30mf2sn non-facing surface, 30mflsm facing surface,
30mf2s1 net-contacting surface (in-contact surface),
30mf2s2 non-net-contacting surface (not-in-contact surface),
31 front band member, 31e end portion,
31s no-absorbent-body portion,
31a front-band-member continuous body,
32 nonwoven fabric sheet, 32B folded portion,
33 nonwoven fabric sheet, 33Le end portion,
35 elastic string (elastic member), 35eu portion
35a elastic-string continuous body (elastic member), 35aed end portion, 35aedn portion,
41 back band member, 41a back-band-member continuous body,
42 nonwoven fabric sheet, 42Le end portion,
43 nonwoven fabric sheet, 43B folded portion,
45 elastic string (elastic member),
45a elastic-string continuous body (elastic member),
50 air-hole forming device (through-hole forming device),
51 upstream roller, 51h hole portion,
55 intermediate roller,
55p pin member (press-in member), 55pa conical portion, 55pb circular column portion,
55r receiving portion, 55rt top surface,
58 downstream roller, 58h hole portion,
60 slitter device,
80 front-side cutter device,
81u cutter roll, 81ua outer circumferential surface,
81d anvil roll, 81da outer circumferential surface,
84 cutter block,
90 back-side cutter device,
201 suction-belt conveyer, 202 net (support member),
202s upper surface (support surface), 204 roller, 206 suction box,
208 nozzle,
211 feeding device, 211a rotation shaft,
215 transporting roller, 215a transporting roller (driven roller),
215D dancer roller, 215S stationary roller,
240 folding device,
h air hole (through hole), C cutter blade, B1 burr, B2 burr,
f fiber, f1 fiber, f2 fiber,
AH stretchy region, AH1 fixed region,
AL non-stretchy region, AL1 non-fixed region,
AU upper region, AD lower region, ADc central region, ADe end region,
BH waist opening, LH leg opening, LG leg gather,
CL1 central position, CL10 predetermined position,
SS side seal portion,
PC predetermined position, TC mark,
BL boundary position, LP loop,
Plsm portion, P1sn portion, P2sm portion, P2sn portion,
PC predetermined position, f1 fiber
C30mf1 material coil, C30mf2 material coil,
Gh31 air hole group, Rh31 air hole row,
Gh41 air hole group, Rh41 air hole row,
G51h1 hole portion group, G51h2 hole portion group,
G55p1 pin member group, G55p2 pin member group,
G58h1 hole portion group, G58h2 hole portion group,
SG51h1 hole portion group set, SG51h2 hole portion group set,
SG55p1 pin member group set, SG55p2 pin member group set,
R51 first transporting route, R55 second transporting route, R58 third transporting route,
P51 closest position (air-hole formation position, through-hole formation position),
P55 closest position,
P230 merging position,
S50 air-hole forming step (through-hole forming step),
S60 slitting step,
S70 gap forming step,
S80 front-elastic-string cutting step,
S90 back-elastic-string cutting step,
S100 absorbent-main-body attaching step,
S110 folding step,
S120 side sealing step,
S130 cutting step,
S200 nonwoven-fabric-sheet producing step,
S210 step at upstream position

## Claims

1. A method for manufacturing a sheet-like member for an absorbent article,
the sheet-like member being formed from a composite sheet that is continuous in a predetermined direction,
the sheet-like member being provided with a plurality of through holes,
the method comprising:
a transporting step of transporting the composite sheet in a direction of transport that is the predetermined direction,
the composite sheet being constituted by a first nonwoven fabric sheet, a second nonwoven fabric sheet, and a plurality of elastic members,
the first nonwoven fabric sheet being continuous in the predetermined direction,
the second nonwoven fabric sheet being continuous in the predetermined direction and arranged overlapped with the first nonwoven fabric sheet in a thickness direction of the composite sheet,
the plurality of elastic members being stretched in the predetermined direction and extending along the predetermined direction,
the plurality of elastic members being inserted between and fixed to the first nonwoven fabric sheet and the second nonwoven fabric sheet in a side-by-side arrangement in a CD direction that intersects the predetermined direction; and
a through-hole forming step of forming the through holes by pressing a press-in member in the thickness direction at positions between a pair of elastic members that are adjacent in the CD direction in the composite sheet,
the through holes penetrating the composite sheet in the thickness direction,
in the through-hole forming step,
the press-in member being pressed in the thickness direction into a non-facing surface of the first nonwoven fabric sheet, and thereafter the press-in member being pulled out toward the non-facing surface,
the non-facing surface being one of two surfaces of the first nonwoven fabric sheet,
the non-facing surface not facing the second nonwoven fabric sheet,
the non-facing surface of the first nonwoven fabric sheet being a surface that comes into contact with a support surface of a support member when constituent fibers of the first nonwoven fabric sheet are suctioned to and deposited on the support surface in order to produce the first nonwoven fabric sheet.

2. The method for manufacturing a sheet-like member for an absorbent article according to claim 1, wherein
a non-facing surface of the second nonwoven fabric sheet is one of two surfaces of the second nonwoven fabric sheet, and does not face the first nonwoven fabric sheet, and
the non-facing surface of the second nonwoven fabric sheet is a surface that comes into contact with a support surface of a support member when constituent fibers of the second nonwoven fabric sheet are suctioned to and deposited on the support surface in order to produce the second nonwoven fabric sheet.

3. The method for manufacturing a sheet-like member for an absorbent article according to claim 1 or 2, wherein
the composite sheet has a fixing portion in which the first nonwoven fabric sheet and the second nonwoven fabric sheet are fixed to each other, and
the through holes are formed in the composite sheet such that the through holes are not overlapped with the fixing portion.

4. The method for manufacturing a sheet-like member for an absorbent article according to any of claims 1 to 3, wherein
the composite sheet has a direct fixing portion in which the first nonwoven fabric sheet and the second nonwoven fabric sheet are fixed to each other without using the elastic members, and
the through holes are formed in the composite sheet such that the through holes are not overlapped with the direct fixing portion.

5. The method for manufacturing a sheet-like member for an absorbent article according to any of claims 1 to 4, wherein
when a surface that is one of two surfaces of the first nonwoven fabric sheet and that is not in contact with the support surface of the support member is defined as a non-contacting surface,
a driven roller is arranged at a position that is upstream, with respect to the direction of transport, of a through hole formation position at which the through holes are formed in the composite sheet in the through-hole forming step, and
the driven roller comes into contact with the non-contacting surface of the first nonwoven fabric sheet being transported, and is thus driven to rotate by rotation force received from the first nonwoven fabric sheet.

6. The method for manufacturing a sheet-like member for an absorbent article according to claim 5, wherein
the driven roller is a dancer roller that is guided to be capable of moving in the predetermined direction in which the first nonwoven fabric sheet is continuous,
a loop is formed in the first nonwoven fabric sheet by the first nonwoven fabric sheet being wound on the dancer roller, and
a predetermined load is applied to the dancer roller in a direction according to which the loop increases in size.

7. The method for manufacturing a sheet-like member for an absorbent article according to any of claims 1 to 6, wherein
the non-facing surface forms a skin-side surface in the absorbent article.

8. The method for manufacturing a sheet-like member for an absorbent article according to any of claims 1 to 6, wherein
the non-facing surface forms a non-skin-side surface in the absorbent article.

9. A device for manufacturing a sheet-like member for an absorbent article,
the sheet-like member being formed from a composite sheet that is continuous in a predetermined direction,
the sheet-like member being provided with a plurality of through holes,
the device comprising:
a transporting device that transports the composite sheet in a direction of transport that is the predetermined direction,
the composite sheet being constituted by a first nonwoven fabric sheet, a second nonwoven fabric sheet, and a plurality of elastic members,
the first nonwoven fabric sheet being continuous in the predetermined direction,
the second nonwoven fabric sheet being continuous in the predetermined direction and arranged overlapped with the first nonwoven fabric sheet in a thickness direction of the composite sheet,
the plurality of elastic members being stretched in the predetermined direction and extending along the predetermined direction,
the plurality of elastic members being inserted between and fixed to the first nonwoven fabric sheet and the second nonwoven fabric sheet in a side-by-side arrangement in a CD direction that intersects the predetermined direction; and
a through-hole forming device that forms the through holes by pressing a press-in member in the thickness direction at positions between a pair of elastic members that are adjacent in the CD direction in the composite sheet,
the through holes penetrating the composite sheet in the thickness direction,
in the through-hole forming device,
the press-in member being pressed in the thickness direction into a non-facing surface of the first nonwoven fabric sheet, and thereafter the press-in member being pulled out toward the non-facing surface,
the non-facing surface being one of two surfaces of the first nonwoven fabric sheet,
the non-facing surface not facing the second nonwoven fabric sheet,
the non-facing surface of the first nonwoven fabric sheet being a surface that comes into contact with a support surface of a support member when constituent fibers of the first nonwoven fabric sheet are suctioned to and deposited on the support surface in order to produce the first nonwoven fabric sheet.

## Patentansprüche

1. Verfahren zur Herstellung eines lagenartigen Elements für einen saugfähigen Artikel,
wobei das lagenartige Element aus einer Verbundlage ausgebildet wird, die in einer vorbestimmten Richtung kontinuierlich ist,
wobei das lagenartige Element über eine Vielzahl von Durchgangslöchern verfügt,
wobei das Verfahren umfasst:
einen Transportschritt zum Transportieren der Verbundlage in eine Transportrichtung, die die vorbestimmte Richtung ist,
wobei die Verbundlage aus einer ersten Faservliesstofflage, einer zweiten Faservliesstofflage und einer Vielzahl von elastischen Elementen ausgebildet wird,
wobei die erste Faservliesstofflage in der vorbestimmten Richtung kontinuierlich ist,
wobei die zweite Faservliesstofflage in der vorbestimmten Richtung kontinuierlich ist und mit der ersten Faservliesstofflage in einer Dickenrichtung der Verbundlage überlappend angeordnet wird,
wobei die Vielzahl von elastischen Elementen in der vorbestimmten Richtung gestreckt ist und sich entlang der vorbestimmten Richtung erstreckt,
wobei die Vielzahl von elastischen Elementen zwischen der ersten Faservliesstofflage und der zweiten Faservliesstofflage in einer nebeneinander liegenden Anordnung in einer CD-Richtung, die die vorbestimmte Richtung schneidet, eingesetzt und an diesen befestigt wird; und
einen Durchgangsloch-Ausbildungsschritt zum Ausbilden der Durchgangslöcher durch Pressen eines Einpresselements in die Dickenrichtung an Positionen zwischen einem Paar elastischer Elemente, die in der CD-Richtung in der Verbundlage angrenzend sind,
wobei die Durchgangslöcher in der Dickenrichtung die Verbundplatte durchdringen,
wobei im Durchgangsloch-Ausbildungsschritt,
das Einpresselement in der Dickenrichtung in eine nicht zugewandte Oberfläche der ersten Faservliesstofflage gepresst wird, und danach das Einpresselement in Richtung der nicht zugewandten Oberfläche herausgezogen wird,
wobei die nicht zugewandte Oberfläche eine von zwei Oberflächen der ersten Faservliesstofflage ist,
wobei die nicht zugewandte Oberfläche nicht der zweiten Faservliesstofflage zugewandt ist,
wobei die nicht zugewandte Oberfläche der ersten Faservliesstofflage eine Oberfläche ist, die mit einer Trägerfläche eines Trägerelements in Kontakt kommt, wenn konstituierende Fasern der ersten Faservliesstofflage an die Trägerfläche angesaugt und auf der Trägerfläche abgelegt werden, um die erste Faservliesbahn herzustellen.

2. Verfahren zur Herstellung eines lagenartigen Elements für einen saugfähigen Artikel gemäß Anspruch 1, wobei
eine nicht zugewandte Oberfläche der zweiten Faservliesstofflage eine von zwei Oberflächen der zweiten Faservliesstofflage ist und nicht der ersten Faservliesstofflage zugewandt ist, und
die nicht zugewandte Oberfläche der zweiten Faservliesstofflage eine Oberfläche ist, die mit einer Trägerfläche eines Trägerelements in Kontakt kommt, wenn konstituierende Fasern der zweiten Faservliesstofflage an die Trägerfläche angesaugt und auf der Trägerfläche abgelegt werden, um die zweite Faservliesstofflage herzustellen.

3. Verfahren zur Herstellung eines lagenartigen Elements für einen saugfähigen Artikel gemäß Anspruch 1 oder 2, wobei
die Verbundlage einen Befestigungsabschnitt aufweist, in dem die erste Faservliesstofflage und die zweite Faservliesstofflage aneinander befestigt werden, und
die Durchgangslöcher in der Verbundlage derart ausgebildet werden, dass die Durchgangslöcher nicht mit dem Befestigungsabschnitt überlappt sind.

4. Verfahren zur Herstellung eines lagenartigen Elements für einen saugfähigen Artikel gemäß einem der Ansprüche 1 bis 3, wobei
die Verbundlage einen direkten Befestigungsabschnitt aufweist, in dem die erste Faservliesstofflage und die zweite Faservliesstofflage ohne Verwendung der elastischen Elemente aneinander befestigt werden, und
die Durchgangslöcher in der Verbundlage derart ausgebildet werden, dass die Durchgangslöcher nicht mit dem direkten Befestigungsabschnitt überlappt sind.

5. Verfahren zur Herstellung eines lagenartigen Elements für einen saugfähigen Artikel gemäß einem der Ansprüche 1 bis 4, wobei
wenn eine Oberfläche, die eine von zwei Oberflächen der ersten Faservliesstofflage ist und nicht mit der Trägerfläche des Trägerelements in Kontakt steht, als eine kontaktlose Oberfläche bestimmt werden,
eine angetriebene Rolle an einer Position angeordnet werden, die in Bezug auf die Transportrichtung stromaufwärts einer Durchgangslochausbildungsposition angeordnet ist, an der die Durchgangslöcher in der Verbundlage im Durchgangsloch-Ausbildungsschritt ausgebildet werden, und
die angetriebene Rolle mit der kontaktlosen Oberfläche der ersten transportierten Faservliesstofflage in Kontakt kommt und somit durch die von der ersten Faservliesstofflage aufgenommene Rotationskraft zum Rotieren angetrieben wird.

6. Verfahren zur Herstellung eines lagenartigen Elements für einen saugfähigen Artikel gemäß Anspruch 5, wobei
die angetriebene Rolle eine Tänzerrolle ist, die derart geführt werden, dass sie sich in der vorbestimmten Richtung bewegen kann, in der die erste Faservliesstofflage kontinuierlich ist,
eine Schlaufe in der ersten Faservliesstofflage ausgebildet wird, indem die erste Faservliesstofflage auf die Tänzerrolle gewickelt wird, und
eine vorbestimmte Last auf die Tänzerrolle in einer Richtung aufgebracht wird, in der die Schleife an Größe zunimmt.

7. Verfahren zur Herstellung eines lagenartigen Elements für einen saugfähigen Artikel gemäß einem der Ansprüche 1 bis 6, wobei
die nicht zugewandte Oberfläche eine hautseitige Oberfläche in dem saugfähigen Artikel ausbildet.

8. Verfahren zur Herstellung eines lagenartigen Elements für einen saugfähigen Artikel gemäß einem der Ansprüche 1 bis 6, wobei
die nicht zugewandte Oberfläche eine nicht hautseitige Oberfläche in dem saugfähigen Artikel ausbildet.

9. Vorrichtung zur Herstellung eines lagenartigen Elements für einen saugfähigen Artikel, ,
wobei das lagenartige Element aus einer Verbundlage ausgebildet ist, die in einer vorbestimmten Richtung kontinuierlich ist,
wobei das lagenartige Element über eine Vielzahl von Durchgangslöchern verfügt,
wobei die Vorrichtung umfasst:
eine Transportvorrichtung, die die Verbundlage in einer Transportrichtung transportiert, die die vorbestimmte Richtung ist,
wobei die Verbundlage aus einer ersten Faservliesstofflage, einer zweiten Faservliesstofflage und einer Vielzahl von elastischen Elementen ausgebildet ist,
wobei die erste Faservliesstofflage in der vorbestimmten Richtung kontinuierlich ist,
wobei die zweite Faservliesstofflage in der vorbestimmten Richtung kontinuierlich ist und mit der ersten Faservliesstofflage in einer Dickenrichtung der Verbundlage überlappend angeordnet ist,
wobei die Vielzahl von elastischen Elementen in der vorbestimmten Richtung gestreckt ist und sich entlang der vorbestimmten Richtung erstreckt,
wobei die Vielzahl von elastischen Elementen zwischen der ersten Faservliesstofflage und der zweiten Faservliesstofflage in einer nebeneinander liegenden Anordnung in einer CD-Richtung, die die vorbestimmte Richtung schneidet, eingesetzt und an diesen befestigt ist; und
eine Durchgangsloch-Ausbildungsvorrichtung, die die Durchgangslöcher durch Pressen eines Einpresselements in die Dickenrichtung an Positionen zwischen einem Paar elastischer Elemente ausbildet, die in der CD-Richtung in der Verbundlage angrenzend sind,
wobei die Durchgangslöcher in der Dickenrichtung die Verbundplatte durchdringen,
wobei in der Durchgangsloch-Ausbildungsvorrichtung,
das Einpresselement in der Dickenrichtung in eine nicht zugewandte Oberfläche der ersten Faservliesstofflage gepresst wird, und danach das Einpresselement in Richtung der nicht zugewandten Oberfläche herausgezogen wird,
wobei die nicht zugewandte Oberfläche eine von zwei Oberflächen der ersten Faservliesstofflage ist,
wobei die nicht zugewandte Oberfläche nicht der zweiten Faservliesstofflage zugewandt ist,
wobei die nicht zugewandte Oberfläche der ersten Faservliesstofflage eine Oberfläche ist, die mit einer Trägerfläche eines Trägerelements in Kontakt kommt, wenn konstituierende Fasern der ersten Faservliesstofflage an die Trägerfläche angesaugt und auf der Trägerfläche abgelegt werden, um die erste Faservliesbahn herzustellen.

## Revendications

1. Procédé de fabrication d'un organe de type feuille pour un article absorbant, l'organe de type feuille étant formé d'une feuille composite qui est continue dans une direction prédéterminée,
l'organe de type feuille étant pourvu d'une pluralité de trous traversants,
le procédé comprenant :
une étape de transport consistant à transporter la feuille composite dans une direction de transport qui est la direction prédéterminée,
la feuille composite étant constituée d'une première feuille d'étoffe non tissée, d'une seconde feuille d'étoffe non tissée, et d'une pluralité d'organes élastiques,
la première feuille d'étoffe non tissée étant continue dans la direction prédéterminée,
la seconde feuille d'étoffe non tissée étant continue dans la direction prédéterminée et agencée chevauchée par la première feuille d'étoffe non tissée dans une direction d'épaisseur de la feuille composite,
la pluralité d'organes élastiques étant étirés dans la direction prédéterminée et s'étendant suivant la direction prédéterminée,
la pluralité d'organes élastiques étant insérés entre et fixés à la première feuille d'étoffe non tissée et à la seconde feuille d'étoffe non tissée dans un agencement côte à côte dans une direction CD qui coupe la direction prédéterminée ; et
une étape de formation de trous traversants consistant à former les trous traversants en enfonçant un organe à enfoncer dans la direction d'épaisseur à des positions entre une paire d'organes élastiques qui sont adjacents dans la direction CD dans la feuille composite,
les trous traversants pénétrant dans la feuille composite dans la direction d'épaisseur, à l'étape de formation de trous traversants,
l'organe à enfoncer étant enfoncé dans la direction d'épaisseur dans une surface non en regard de la première feuille d'étoffe non tissée, et ensuite l'organe à enfoncer étant sorti vers la surface non en regard,
la surface non en regard étant l'une de deux surfaces de la première feuille d'étoffe non tissée,
la surface non en regard n'étant pas en regard de la seconde feuille d'étoffe non tissée,
la surface non en regard de la première feuille d'étoffe non tissée étant une surface qui vient en contact avec une surface de support d'un organe de support lorsque des fibres constitutives de la première feuille d'étoffe non tissée sont aspirées et déposées sur la surface de support afin de produire la première feuille d'étoffe non tissée.

2. Procédé de fabrication d'un organe de type feuille pour un article absorbant selon la revendication 1, dans lequel
une surface non en regard de la seconde feuille d'étoffe non tissée est l'une de deux surfaces de la seconde feuille d'étoffe non tissée, et ne fait pas face à la première feuille d'étoffe non tissée, et
la surface non en regard de la seconde feuille d'étoffe non tissée est une surface qui vient en contact avec une surface de support d'un organe de support lorsque des fibres constitutives de la seconde feuille d'étoffe non tissée sont aspirées et déposées sur la surface de support afin de produire la seconde feuille d'étoffe non tissée.

3. Procédé de fabrication d'un organe de type feuille pour un article absorbant selon la revendication 1 ou 2, dans lequel
la feuille composite a une portion de fixation dans laquelle la première feuille d'étoffe non tissée et la seconde feuille d'étoffe non tissée sont fixées l'une à l'autre, et
les trous traversants sont formés dans la feuille composite de sorte que les trous traversants ne sont pas chevauchés par la portion de fixation.

4. Procédé de fabrication d'un organe de type feuille pour un article absorbant selon l'une quelconque des revendications 1 à 3, dans lequel
la feuille composite a une portion de fixation directe dans laquelle la première feuille d'étoffe non tissée et la seconde feuille d'étoffe non tissée sont fixées l'une à l'autre sans utiliser les organes élastiques, et
les trous traversants sont formés dans la feuille composite de sorte que les trous traversants ne sont pas chevauchés par la portion de fixation directe.

5. Procédé de fabrication d'un organe de type feuille pour un article absorbant selon l'une quelconque des revendications 1 à 4, dans lequel
lorsqu'une surface qui est l'une de deux surfaces de la première feuille d'étoffe non tissée et qui n'est pas en contact avec la surface de support de l'organe de support est définie en tant que surface sans contact,
un rouleau entraîné est agencé à une position qui est en amont, par rapport à la direction de transport, d'une position de formation de trous traversants à laquelle les trous traversants sont formés dans la feuille composite à l'étape de formation de trous traversants, et
le rouleau entraîné vient en contact avec la surface sans contact de la première feuille d'étoffe non tissée qui est transportée, et est ainsi entraîné en rotation par une force de rotation reçue de la première feuille d'étoffe non tissée.

6. Procédé de fabrication d'un organe de type feuille pour un article absorbant selon la revendication 5, dans lequel
le rouleau entraîné est un rouleau danseur qui est guidé pour être capable de se déplacer dans la direction prédéterminée dans laquelle la première feuille d'étoffe non tissée est continue,
une boucle est formée dans la première feuille d'étoffe non tissée par l'enroulement de la première feuille d'étoffe non tissée sur le rouleau danseur, et
une charge prédéterminée est appliquée au rouleau danseur dans une direction selon laquelle la boucle augmente de taille.

7. Procédé de fabrication d'un organe de type feuille pour un article absorbant selon l'une quelconque des revendications 1 à 6, dans lequel
la surface non en regard forme une surface côté peau dans l'article absorbant.

8. Procédé de fabrication d'un organe de type feuille pour un article absorbant selon l'une quelconque des revendications 1 à 6, dans lequel
la surface non en regard forme une surface non côté peau dans l'article absorbant.

9. Dispositif de fabrication d'un organe de type feuille pour un article absorbant, l'organe de type feuille étant formé d'une feuille composite qui est continue dans une direction prédéterminée,
l'organe de type feuille étant pourvu d'une pluralité de trous traversants,
le dispositif comprenant :
un dispositif de transport qui transporte la feuille composite dans une direction de transport qui est la direction prédéterminée,
la feuille composite étant constituée d'une première feuille d'étoffe non tissée, d'une seconde feuille d'étoffe non tissée, et d'une pluralité d'organes élastiques,
la première feuille d'étoffe non tissée étant continue dans la direction prédéterminée,
la seconde feuille d'étoffe non tissée étant continue dans la direction prédéterminée et agencée chevauchée par la première feuille d'étoffe non tissée dans une direction d'épaisseur de la feuille composite,
la pluralité d'organes élastiques étant étirés dans la direction prédéterminée et s'étendant suivant la direction prédéterminée,
la pluralité d'organes élastiques étant insérés entre et fixés à la première feuille d'étoffe non tissée et à la seconde feuille d'étoffe non tissée dans un agencement côte à côte dans une direction CD qui coupe la direction prédéterminée ; et
un dispositif de formation de trous traversants qui forme les trous traversants en enfonçant un organe à enfoncer dans la direction d'épaisseur à des positions entre une paire d'organes élastiques qui sont adjacents dans la direction CD dans la feuille composite,
les trous traversants pénétrant dans la feuille composite dans la direction d'épaisseur, dans le dispositif de formation de trous traversants,
l'organe à enfoncer étant enfoncé dans la direction d'épaisseur dans une surface non en regard de la première feuille d'étoffe non tissée, et ensuite l'organe à enfoncer étant sorti vers la surface non en regard,
la surface non en regard étant l'une de deux surfaces de la première feuille d'étoffe non tissée,
la surface non en regard n'étant pas en regard de la seconde feuille d'étoffe non tissée,
la surface non en regard de la première feuille d'étoffe non tissée étant une surface qui vient en contact avec une surface de support d'un organe de support lorsque des fibres constitutives de la première feuille d'étoffe non tissée sont aspirées et déposées sur la surface de support afin de produire la première feuille d'étoffe non tissée.
